# EUROPEAN PATENT SPECIFICATION

(11) **EP 3 801 531 B1**
(45) Date of publication and mention of the grant of the patent: **18.09.2024**
(21) Application number: 19820488.5
(22) Date of filing: 10.06.2019
(51) Int. Cl.: A61K 31/506, A61K 9/00, A61K 9/48, A23G 3/36, A23G 3/42

(54) **LOW GLYCEMIC GUMMY COMPOSITION AND METHODS OF MAKING AND USING THEREOF**
GUMMIZUSAMMENSETZUNGEN MIT NIEDRIGEM GLYKÄMISCHEM INDEX UND VERFAHREN ZUR HERSTELLUNG UND VERWENDUNG DAVON
COMPOSITION GOMMEUSE À FAIBLE INDICE GLYCÉMIQUE ET SES PROCÉDÉS DE FABRICATION ET D'UTILISATION

(30) Priority: 11.06.2018 US 201862683523 P
(43) Date of publication of application: 14.04.2021
(73) Proprietor: Seattle Gummy Company, Seattle, Washington 98104 (US)
(72) Inventor: WAN, Feng, Issaquah, Washington 98029 (US); CARLSON, William, Shoreline, Washington 98155 (US)
(74) Representative: Hobson, David James
(86) International application number: PCT/US2019/036370
(87) International publication number: WO 2019/241146

(56) References cited:
- EP-A1- 3 269 251
- EP-B1- 2 945 492
- WO-A1-2015/075473
- WO-A1-2016/135458
- WO-A1-2017/059352
- WO-A1-2017/081667
- WO-A1-2017/081667
- WO-A1-2017/144486
- WO-A1-2018/029698
- WO-A1-2019/140403
- CA-A- 1 051 260
- US-A1- 2003 044 503
- US-A1- 2006 003 965
- US-A1- 2006 034 993
- US-A1- 2010 267 658
- US-A1- 2014 342 074
- US-A1- 2015 351 422
- US-A1- 2016 296 470
- US-A1- 2016 302 463
- "The technology of wafers and waffles (Volume I) : Operational aspects", 18 May 2017, ELSEVIER, US, article KARL F. TIEFENBACHER: "Technology of Main Ingredients?Sweeteners and Lipids: 3.2 TECHNOLOGY OF OILS AND FATS FOR WAFERS AND WAFFLES 3.2.1 Introduction", pages: 182 - 215, XP055768404, DOI: 10.1016/B978-0-12-809438-9.00003-X
- KARL F TIEFENBACHER: "The technology of wafers and waffles (Volume I) : Operational aspects", 18 May 2017, ELSEVIER, US, ISBN: 978-0-12-809438-9, article KARL F TIEFENBACHER: "Passage; Technology of Main Ingredients—Sweeteners and Lipids: 3.1 Technology of sugars , syrups , sugar replacements and sweeteners ", pages: 182, XP009525167, DOI: 10.1016/C2015-0-04250-2
- MARESCH ET AL.: "Low glycemic index prototype isomaltulose?update of clinical trials", NUTRIENTS, vol. 9.4, 13 April 2017 (2017-04-13), pages 381, XP055555011

## Description

### TECHNICAL FIELD

The application relates generally to a nutraceutical composition in gelled or gummy formula, methods of administration of various gummy or gelled compositions for bioactive compounds, and kits comprising various gummy compositions.

### BACKGROUND

Current gummy confectioneries and products are made with mostly simple sugars, for example, as much as 80% by weight of a traditional gummy composition is comprised of simple sugars. The most common sugars used in gummy composition are sucrose and glucose. Sucrose and glucose are simple mono and disaccharides with high glycemic index (GI) value. For example, sucrose has a GI value of 63 and glucose has GI value of 100. These sugars can lead to a spike in glucose levels. Consequently, simple sugars, such as glucose and sucrose, are not tolerated well by population that are either diabetics or with diabetic tendency.

Previous technologies have utilized non-sugar sweeteners (sugar substitutes) to reduce the amount of sugar utilized in gummy confectioneries. Conventional sugar substitutes used include sugar alcohol (such as erythritol, lactitol, maltitol, sorbitol, xylitol, isomalt and mannitol) and non-sugar sweeteners (such as sucralose and aspartame). The conventional sugar substitutes are often not digestible by the body or are poorly absorbed. Consequently, the conventional sugar substitutes change the osmotic pressure within the digestive track when reaching the colon, causing the colon to absorb water, leading to gastric distress and bloating, gas, or diarrhea. In addition, non-sugar sweeteners tend to have un-natural sweet taste.

There is a need for a gummy product that is low in glycemic index, naturally sweet tasting while not causing gastric distress.

WO 2018/029698 A1 relates to a sweetener composition comprising Glucoseoxidase enzyme (GOD). The composition further includes at least one low glycemic index nutritive ingredient with a glycemic index of less than 70.

EP 3 269 251 A1 relates to short texture caramel containing bimodal carbohydrate ingredients that combine high molecular weight polysaccharides and at least one monosaccharide.

WO 2017/081667 A1 relates to a low calorie, low glycemic index (GI), and sustained energy release sugar composition comprising a combination of isomaltulose, trehalulose and D-allulose; at least one of the following: essential trace elements, soluble oligosaccharides and bulking agents; and optionally, one or more nutritive sweetener for use in a food and beverage product.

Karl F. Tiefenbacher "Technology of Main Ingredients-Sweeteners and Lipids: 3.2 Technology of oils and fats for wafers and waffles 3.2.1 Introduction In: "The Technology of wafers and waffles (Volume I): Operational aspects", 18 May 2017 relates to the technology of oil and fat ingredients, their right handling, and potential technological and nutritional issues.

US 2014/342074 A1 relates to a sweetener composition for alleviating diabetes, containing psicose and a slowly digestible or digestion-resistant polysaccharide as active ingredients, wherein diabetes alleviating effects and the quality of sweetness are enhanced.

WO 2017/059352 A1 relates to a low calorie, low laxation confection such as chewy candy, hard candy, tableted candy, or gelled candy having acceptable texture, stability, clarity, and flavor delivery that contains a bulk sweetener comprising allulose (psicose).

WO 2015/075473 A1 relates to the use of high levels of allulose in food and beverage products.

WO 2016/135458 A1 relates to allulose syrups, use of allulose syrups in the manufacture of food or beverage products, and food and beverage products made using the allulose syrups.

EP 2 945 492 B1 relates to jelly confectionary such as gummy bears, in particular to jelly confectionary, which is non-cariogenic, non-laxative, reduced-calorie, and has a low glycemic index.

CA 1 051 260 A relates to a method of preparing sweetened foods free of cariogenic properties, which comprises adding to the foods sorbose as sole sweetening agent.

WO 2017/144486 A1 relates to a method of preparation of chewy candies comprising branched maltodextrins and crystalline allulose particles in such a way to maintain chewiness, to bring the "body" of the chew, to limit sandiness, to avoid hardening with time and to lower stickiness textures, and even in such away to avoid cold flow.

WO 2007/061753 A2 relates generally to functional sweetener compositions comprising non-caloric or low-caloric natural and/or synthetic high-potency sweeteners and methods for making and using them.

WO 2007/061911 A2 relates generally to edible gel compositions comprising non-caloric or low-caloric high-potency sweeteners and methods for making and using them.

WO 2019/140403 A1 relates to a semi-solid composition, comprising a gelling component in a sufficient amount to provide a cohesive gelled product, an anti-histamine composition, comprising an antihistamine, and a complexing agent, wherein the complexing agent is capable of interacting with the antihistamine and forming an antihistamine complex.

### SUMMARY

Naturally sweet tasting gummy compositions having a low glycemic index as claimed are provided. The invention provides a gummy composition comprising a gelling component in a sufficient amount to provide a cohesive gelled product, and a low glycemic index sugar component having a glycemic index of not more than 35, wherein the gummy composition comprises not less than 50% weight of the low glycemic index sugar component, and wherein the gummy composition is substantially free of D-glucose, D-frucose, D-sucrose, and sugar alcohols, wherein the low glycemic index sugar component comprises trehalose, isomaltulose and psicose.

The invention also provides a process for preparing a gummy composition of the invention, comprising,
mixing a gelling agent with the active component in hot water to provide a first solution,
mixing low GI sugars in hot water to provide a second solution,
combining the first solution with the second solution to provide a total mixture, wherein the total mixture is heated to brix 82 to provide a gummy base, and
adding food acid into the gummy base to provide a gummy composition.

In one embodiment, the low glycemic index sugar component has a glycemic index of not more than 30. In one embodiment, the low glycemic index sugar component has a glycemic index of not more than 20. In one embodiment, the low glycemic index sugar component has a glycemic index of not more than 10. In one embodiment, the low glycemic index sugar component is not less than 60% weight of the gummy composition. In one embodiment, the low glycemic index sugar component is not less than 70% weight of the gummy composition. In one embodiment, the low glycemic index sugar component is from about 70% to about 75% weight of the gummy composition.

In one embodiment, the low glycemic index sugar component comprises a low glycemic index sugar having a glycemic index of not more than 35, 30, 20, 15, 10, or 8 and a molecular weight of not more than 540. In one embodiment, the low glycemic index sugar component consists essentially of low glycemic index sugars, wherein each low glycemic index sugar has a glycemic index of not more than 35, 30, 20, 15, 10, or 8 and a molecular weight of not more than 360.

In one embodiment, the low glycemic index sugar component comprises a low GI monosaccharide, a low GI disaccharide, a low GI tri-saccharide, or a combination thereof. In one embodiment, the low GI monosaccharide comprises a stereoisomer of D-fructose, D-glucose, or D-galactose. In one embodiment, the low GI monosaccharide comprises L-fructose, L-glucose, L-galactose, Psicose (D- or L-), Sorbose (D- or L-), Tagatos (D- or L-)e, or a combination thereof. In one embodiment, the low GI disaccharide comprises an isomer of D-maltose (1,4- diglucose) or an isomer of D-sucrose (1,2- fructose glucose). In one embodiment, the low GI disaccharide comprises trehalose, D- or L-isomaltulose (isomaltuose), or a combination thereof. In one embodiment, the low GI tri-saccharide comprises raffinose (D- or L-). The low GI sugar component comprises trehalose and isomaltulose. According to the invention, the low GI sugar component comprises trehalose, isomaltulose, and psicose.

In one embodiment, the low glycemic index sugar component may include psicose (allulose) by a weight of not less than 30%, 40%, 50%, 55%, 60% or 70%. In one embodiment, the low glycemic index sugar component may include psicose (allulose) by a weight from about 50% to about 60%.

In one embodiment, the low glycemic index sugar component may include trehalose by a weight of not less than 15%, 20%, 22%, 22.5%, 25%, 30%, 40%, 50%, 55%, or 60%. In one embodiment, the low glycemic index sugar component may include trehalose by a weight from about 20% to about 30%.

In one embodiment, the low glycemic index sugar component may include isomaltulose by a weight of not less than 5%, 10%, 15%, 20%, 22%, 22.5%, 25%, 30%, 40%, 50%, 55%, or 60%. In one embodiment, the low glycemic index sugar component may include isomaltulose by a weight from about 10% to about 30%.

In one embodiment, the low glycemic index sugar component may include trehalose and isomaltulose in a ratio from about 3:1 to about 1:3. In one embodiment, the low glycemic index sugar component may include trehalose and isomaltulose in a ratio of 3: 1, 2:1, 1:1, 1:2, 2:1, 3:1.

In one embodiment, the low GI sugar component further comprises N-acetyl glucosamine. In one embodiment, the gummy composition comprises less than 0.001%, 0.1% or 1% of non-sugar sweeteners, sugar substitutes, or sugar alcohols. In one embodiment, the gummy composition is substantially free of D-sucrose, D-fructose, D-maltose, or D-glucose. According to the invention, the gummy composition is substantially free of D-glucose, D-fructose, D-sucrose, and sugar alcohols. In one embodiment, the gummy composition has a natural sweetness taste profile substantially similar to D-sucrose.

In some embodiments, the gummy composition may have a glycemic index value of not more than 20. In some embodiment, the gummy composition may have a glycemic index value from about 0 to about 15. In some embodiments, the gummy composition may have glycemic index of not more than 15, 10, 8, 6 or 5. In some embodiment, the gummy composition may have glycemic index of about 0.

Low glycemic index (GI) sugars are used in the disclosed gummy compositions. The low GI sugars useful in the application may be, without limitation, mono-saccharides, disaccharides, tri-saccharides, or tetra-saccharides. In some embodiments, low GI sugars useful in this application may be mono-saccharides, di-saccharides or tri-saccharides having a GI value of not exceeding 20. In some embodiments, the low GI sugars may have a glycemic index value of not more than 12. In some embodiments, the low GI sugars may have a glycemic index value of not more than 10, 9, 8, 7, 6, or 5. In some embodiments, the low GI sugars may have a glycemic index value of about 0.

The low GI sugars used in this application are saccharides chemically; therefore they are not sugar substitutes or artificial sweeteners. In some embodiments, the low GI sugars are saccharides containing hydrogen and oxygen in the same ratio as water (2:1). In one embodiment, the low GI sugar comprises from about 1 to about 5 monosaccharides. In some embodiments, the low GI sugars have a molecule weight of not less than 90. In some embodiments, the low GI sugars have a molecule weight of not exceeding 505. Examples of the low GI sugars include, without limitation, L-glucose, L-sucrose, L-galactose, D- or L-isomaltulose, D- or L-trehalose, D- or L-psicose, D- or L-tagatose, and D- or L-sorbose. According to the invention, the low glycemic index sugar component comprises trehalose, isomaltulose and psicose.

In one embodiment, the gummy composition may further comprise glucosamine. In one embodiment, the gummy composition may further comprise isomalt.

In some embdoiments, the gummy compositions comprise not less than 68% weight of low GI sugars. In some embodiments, the gummy compositions comprise not less than 60%, 70%, 80%, 90%, or 99% weight of low GI sugars. The percentage can be any amount in between the ranges recited herein. According to the invention, the gummy composition comprises not less than 50% weight of the low glycemic index sugar component.

In one embodiment, the gummy composition may be substantially free of sugar substitutes. In one embodiment, the gummy composition may be substantially free of artificial sweeteners. According to the invention, the gummy compositions are substantially free of sugar alcohols.

In some embodiments, the low GI sugars may be fully or partially absorbable in the digestive tract. In some embodiments, low GI sugars may digest very slowly in the body and have minimal impact on blood sugar levels. Some low GI sugars may be absorbed by the body but are not metabolized. In some embodiments, the low GI sugars are not readily digested or metabolized by the body. In some embodiments, the low GI sugars are primarily excreted in the urine. As a result, these sugars do not lead to the gastric distress that the sugar alcohols may cause. In some embodiments, the gummy composition is configured to not causing gastric distress that the sugar alcohols may cause.

Due to the surprising synergistically enhanced sweetness when combining the low GI sugars disclosed herein, the disclosed gummy compositions are sweet like conventional sugar gummy compositions, unlike conventional sugar substitutes, which often impart "unnatural," "tacky," "metallic," or "artificial" sweet taste often with lingering, "sticky," or "bitter" after taste. The gummy composition disclosed herein retains a natural flavor and sweetness (with a sweetness taste profile similar to sucrose) that the conventional sugar substitutes lack. In one embodiment, the gummy composition provides a sucrose equivalent value (SEV) greater than the predicted value based on its individual components. In one embodiment, the gummy compositions provide a substantially similar temporal profile as a conventional gummy product made with sucrose, glucose, fructose or a combination thereof. In one embodiment, the gummy compositions provide a substantially similar sweetness profile as a conventional gummy product made with sucrose, glucose, fructose or a combination thereof.

In one embodiment, the gelling component comprises gelatin, collagen, starch, pectin, gellan gum, gum Arabic, carrageenans, guar, agar, alginate, locust bean gum, xanthan, or derivatives thereof. In one embodiment, the starch comprises corn starch, tapioca starch, potato starch, wheat starch, dextrins, maltodextrins, thin-boiling starch, high amylose corn starch, instant starches, or derivatives thereof.

In one embodiment, the gummy composition comprises about 0.1% to about 5% by weight pectin based on the total weight of the composition. In one embodiment, the gummy composition comprises about 0.5% by weight pectin to about 6% pectin and about 2% to about 10% by weight gelatin based on the total weight of the composition. In one embodiment, the gummy composition comprises about 0.5% by weight pectin to about 6% pectin and about 1% to about 12% by weight starch based on the total weight of the composition.

In one embodiment, the gummy composition is essentially free of gelatin. In one embodiment, the gummy composition comprises less than 10%, 5%, 3%, 2%, 1%, 0.5%, 0.2% weight of the gelling component. In one embodiment, the gelling component consists essentially of pectin and gelatin. In one embodiment, the gelling component consists essentially of pectin and collagen.

In one embodiment, the gummy composition may further comprises an active component, wherein the active component comprises a vitamin composition, a mineral composition, an amino acid composition, an antioxidant composition, an herbal composition, or an active pharmaceutical ingredient (API) composition.

In one embodiment, the gummy composition comprises less than 50%, 20%, 10%, 5%, 2%, 1% or 0.8% of the active component. In one embodiment, the gummy composition comprises more than 0.01%, 0.1%, 0.5%, 0.7%, 0.8%, 1%, 1.5%, 2%, 5%, or 10% of the active component.

In one embodiment, the vitamin composition comprises vitamin A, vitamin B, vitamin C, vitamin D, vitamin E, vitamin K, or a derivative thereof. In one embodiment, the vitamin B comprises Vitamin B1 (thiamine), Vitamin B2 (riboflavin), niacin, vitamin B6, vitamin B12, folate, pantothenic acid, biotin, or derivatives thereof.

In one embodiment, the mineral composition comprises salts or chelates of calcium, iron, zinc, magnesium, sodium, chloride, potassium, copper, molybdenum, manganese, phosphorus, iodine, nickel, or selenium, selenium yeast, or a combination thereof. In one embodiment, the chelates are amino acid chelates.

In one embodiment, the amino acid composition comprise leucine, isoleucine, valine, arginine, glutamine, alanine, carnitine, creatine, tryptophan, aspartate, ornithine, tyrosine, taurine, lysine, histidine, threonine, phenylalanine, methionine, or any derivative thereof. In one embodiment, the amino acid composition comprises leucine, isoleucine, valine and a combination thereof. In one embodiment, the amino acid composition comprises leucine, isoleucine, and valine at a ratio of 2: 1: 1.

In one embodiment, the antioxidant composition comprises Vitamin A, Vitamin E, Vitamin C, selenium, selenium yeast, polyphenols, phenolics, anthocyanins, flavonoids, anthracenes, ubiquinone, carotenoid terpenoids, or derivatives thereof. In one embodiment, the antioxidant composition comprises retinol, ascorbic acid, vitamin E, coenzyme Q10, manganese, iodide, melatonin, alpha-carotene, astaxanthin, beta-carotene, canthazanthin, lutein, lycopene, zeaxanthin, apigenin, luteolin, tangeritin, isorhamnetin, kaempferol, myricetin, proanthocyanidins, quercetin, rutin, eriodictyol, hesperetin, neringenin, catechin or corresponding gallate esters, gallocatechin or corresponding gallate esters, epicatechin or its gallate ester, epigallocatechin or its gallate ester, theaflavin and its gallate esters, thearubigins, daidzein, genistein, glycitein, resveratrol, pterostilbene, anthocyanins, delphinidin, malvidin, pelargonidin, peonidin, petunidin, chicoric acid, chlorogenic acid, cinnamic acid, ferulic acid, ellagic acid, ellagitannins, rosmarinic acid, salicylic acid, curcumin, flavonolignans, silymarin, xanthones, mangosteen, capsaicin, bilirubin, citric acid, oxalic acid, phytic acid, N-acetylcysteine, R-alpha-lipoic acid, glutathione, or their derivatives thereof.

In one embodiment, the herbal composition comprises Angelica sinesis, Astragalus, red dates, goji berry, longan berry, Condonoposis Pilosula (Dangshen), ginseng, gandoerma (Linzhi), coix seed, ginger, Bletilla Tuber (Baiji), white Atractylodes rhizome (Baishu), Radix Angelicae Dahuricae (Baizhi), Typhonium Tuber (baifuzi), Radix ampelopsis (bailian), Rhizoma Bletillae (baiji), Rhizoma Polygonati Odorati (yuzhu), Adenophora root (shashen), lily bulb (baihe), mune bean, licorice, ampelopsis root (bailian), white peony root (baishao), Chinese Yam (Rhizoma Dioscoreae, shanyao), poria (fulin), schisandra, mint, aloe, lemon peel, lemon, orange peel, Polygonum Multiflorum (he-shou-wu), ginko, turmeric, gotu kola (*centella asiatica*), ashwagandha, *tribulus terrestris, mucunna pruriens,* guarana, mate (*Ilex paraguariensis*)*,* ephedra (ephedra sinica or ma-huang), ciwujia, rose, lotus, honeysuckle, chrysanthemum, osmanthus fragran, jasmine, dandelion, peach blossom, blueberry, acai berry, raspberry, blackberry, huckleberry, cranberry, strawberry, lingonberry, gooseberry, black or red currants, cloudberry, hawthorn berry, coffee, maca, or powder, extracts or isolates thereof.

In one embodiment, the herbal composition comprises powder, extract, or isolates of a herb, wherein the herb comprises a plant seed, fruit, flower, root, leaf, stem, or a combination thereof. In one embodiment, the herbal composition comprises ginger, turmeric, ginseng, angelica sinesis, astragalus, goji berry, or its extract or isolate thereof. In one embodiment, the ginseng comprises Asian ginseng (panax ginseng), Siberian ginseng (Eleutherococcus senticosus), American ginseng (Panax quinquefolius), indian ginseng (Withania somnifera), or a combination thereof.

In one embodiment, the API composition comprises an antibacterial agent, an antifungal agent, a pain or fever reliever, an analgesics, an anti-inflammatory agent, a steroid, a diabetic medication, an antidiarrheal agent, an antiulcer agent, a proton pump inhibitor, a laxative or cathartic agent, a diuretic agent, an antacid, an antihistamine, a decongestant, an antitussive or expectorant agent, a cough suppressant, a cold medicine, a motion sickness medication, a medication for treating alcoholism or opioid dependence, a smoke cessation agent, an anti-anxiety agent, an antidepressant, a mood stabilizer, an antipsychotic agent, a stimulant, a benzodiazepine, an anxiolytic agent, a Z-drug, a melatonergic agent, a barbiturate, a antidepressant, an antipsychotic agent, a cardiovascular agent, an anti-cancer agent, an immune suppressant, a blood thinner, or a combination thereof.

In one embodiment, the antibacterial agent comprises a penicillin, a cephalosporin, a macrolide, a fluroquinolone, a sulfonamide, a tetracycline, an aminoglycoside, or a combination thereof. In one embodiment, the antibacterial agent comprises bacitracin, clotrimazole, miconazole, penicillin, amoxicillin, cephalexin, erythromycin, clarithromycin, azithromycin, ciprofloxacin, levofloxacin, ofloxacin, co-trimoxazole, trimethoprim, tetracycline, doxycycline, gentamicin, tobramycin, or a combination thereof.

In one embodiment, the pain or fever reliever comprises acetaminophen, aspirin, salicylic acid, ibuprofen, naproxen, or a combination thereof.

In one embodiment, the antidiarrheal agent comprises loperamide, or a combination thereof.

In one embodiment, the antiulcer agent comprises cimetidine, faniotidine, nizatidine, ranitidine, misoprostol, sucralfate or a combination thereof.

In one embodiment, the proton pump inhibitor comprises omeprazole, esomeprazole, rabeprazole, pantoprazole, lansoprazole, dexlansoprazole or a combination thereof.

In one embodiment, the laxative or cathartic agent comprises bisacodyl, docusate, miralax, psyllium, senokot, castor oil, magnesium hydroxide, magnesium citrate, magnesium sulfate, lactulose or a combination thereof.

In one embodiment, the antihistamine comprises cetirizine, diphenhydramine, loratadine, chlorpheniramine, brompheniramine, alimemazine, cyprohetadine, doxylamine, hydroxyzine, promethazine, or a combination thereof.

In one embodiment, the decongestant comprises pseudoephedrine, oxymetazoline, phenylephrine or a combination thereof.

In one embodiment, the antitussive and expectorant comprises guaifenesin, codeine phosphate, dextromethorphan hydrobromide, or a combination thereof.

In one embodiment, the cough suppressant comprises dextromethorphan.

In one embodiment, the medication for treating alcoholism or opioid dependence comprises acamprosate, baclofen, buprenorphine, naloxone, clonidine, disulfiram, methadone, naltrexone, ondansetron, or a combination thereof.

In one embodiment, the smoke cessation agent comprises nicotine, bupropion, cytosine, varenicline, or a combination thereof.

In one embodiment, the mood stabilizer comprises carbamazepine, gabapentin, lamotrigine, levetiracetam, lithium salt, oxcarbazepine, topiramate, sodium valproate, divalproex sodium, valproic acid,

In one embodiment, the antipsychotic agent comprises aripiprazole, asenapine, chlorpromazine, olanzapine, paliperidone, quetiapine, risperidone, ziprasidone,

In one embodiment, the stimulant comprises amphetamine, dexamfetamine, lisdexamfetamine, methylphenidate, dexmethylphenidate, metanifetamine, or a combination thereof.

In one embodiment, the benzodiazepine comprises alprazolam, bromazepam, chlordiazepoxide, clobazam, clonazepam, clorazepate, diazepam, lorazepam, oxazepam, tofisopam, brotizolam, estazolam, flunitrazepam, flurazepam, loprazolam, lormetazepam, midazolam, nimetazepam, nitrazepam, phenazepam, quazepam, temazepam, triazolam, or a combination thereof.

In one embodiment, the anxiolytic agent comprising buspirone, hydroxyzine, meprobamate, pregabalin, or a combination thereof.

In one embodiment, the Z-drug comprises eszopicolone, zaleplon, zolpidem, zopiclone, or a combination thereof.

In one embodiment, the melatonergic agent comprises agomelatine, ramelteon, or a combination thereof.

In one embodiment, the barbiturate comprises amobarbital, secobarbital, butobarbital, cyclobarbital, diazepam, pentobarbital, phenobarbital, secobarbital, or a combination thereof.

In one embodiment, the antidepressant comprises citalopram, clomipramine, dexopin, escitalopram, fluoxetine, fluvoxamine, imipramine, mirtazapine, paroxetine, sertraline, trazodone, amitriptyline, doxepin, mianserin, mirtazapine, trazodone, trimipramine, or a combination thereof.

In one embodiment, the antipsychotic agent comprises chlorprothizene, levomepromazine, perazine, promethazine, prothipendyl, sulpiride, thioridazine, zuclopenthixol, perphenazine, beneperidol, bromperidol, fluphenazine, fluspirilen, haloperidol, pimozide, amisulpride, aripiprazole, asenapine, chloropromazine, clozapine, flupenthixol, lloperidone, melperone, olanzapine, paliperidone, penfluridol, quetiapine, reserpine, risperidone, sertindole, thiothizene, trifluoperazine, zipraisdone, zotepine, pericyazine, carbamazepine, valproic acid, or a combination thereof.

In one embodiment, the cardiovascular agent comprises an aldosterone receptor antagonist, an angiotensin converting enzyme inhibitor, an angiotensin receptor blocker, a neprilysin inhibitor, an antiadrenergic agent, an antianginal agent, an antiarrhythmic agent, an anticholinergic chronotropic agent, an antihypertension agent, an ACE inhibitor, an angiotensin II inhibitor, an antiadrenergic agent, a beta blocker, a diuretic agent, a beta-adrenergic blocker, a calcium channel blocker, a catecholamine, an inotropic agent, a vasodilator, a renin inhibitor, a sclerosing agent, a vasopressin antagonist, a vasopressor, a anti-cholesterol agent, or a combination thereof.

In one embodiment, the antihypertension agent comprises diazoxide, fenoldopam, nitroprusside, a thiazide, or a combination thereof.

In one embodiment, the API composition comprises loratidine, cetirizine, fexofenadine, diphenhydramine, aspirin, acetaminophen, ibuprofen, dextromethorphan, pseudoephedrine, naproxen, avanafil, tadalafil, sildenafil, vardenafil, alprostadil, caffeine, or a derivative thereof.

In one embodiment, the gummy composition may further comprise an additive. In one embodiment, the additive comprises sweeteners, food acids, flavoring agents, coloring agents, humectants, bulking agents, fatty acids, triglycerides, emulsifiers, thickeners, cyclodextrin, or and a mixture thereof.

In one embodiment, the gummy composition may further comprise a sweetener. In one embodiment, an artificial sweetener is selected from the group consisted of saccharin, saccharin salts, cyclamic acid, cyclamic acid salts, aspartame, sucralose, acesulfame, and combinations thereof.

In one embodiment, the gummy composition may further comprises a sweetener selected from the group consisting of rebaudioside A, rebaudioside B, rebaudioside C, rebaudioside D, rebaudioside E, dulcoside A, dulcoside B, rubusoside, stevia, stevioside, mogroside IV, mogroside V, Luo Han Guo (monk fruit) sweetener, thaumatin, katemfe fruit extract, siamenoside, monatin and its salts (monatin SS, RR, RS, SR), curculin, glycyrrhizic acid and its salts, thaumatin, monellin, mabinlin, brazzein, hemandulcin, phyllodulcin, glycyphyllin, phloridzin, trilobatin, baiyunoside, osladin, polypodoside A, pterocaryoside A, pterocaryoside B, mukurozioside, phlomisoside I, periandrin I, abrusoside A, cyclocarioside I, sucralose, acesulfame potassium and other salts, aspartame, alitame, saccharin, neohesperidin dihydrochalcone, cyclamate, neotame, N-[N-[3-(3-hydroxy-4- methoxyphenyl)propyl]-L-.alpha.-aspartyl]-L- -phenylalanine 1-methyl ester, N-[N-[3-(3-hydroxy-4- methoxyphenyl)-3-methylbutyl]-L-. alpha. -aspartyl]-L-- phenylalanine 1-methyl ester, N-[N-[3-(3-methoxy- 4-hydroxyphenyl)propyl]-L-.alpha.-aspartyl]-L-phenylal- anine 1-methyl ester, salts thereof, and combinations thereof. In one embodiment, the gummy composition may further comprise a sweetener comprising erythritol, xylitol, or derivatives or combinations thereof.

In one embodiment, the gummy composition further comprises a food acid component. In one embodiment, the food acid component comprises malic acid, fumaric acid, lactic acid, tartaric acid, glucono-delta lactone, salts of gluconic acid, phosphoric acid, succinic acid, adipic acid, acetic acid, citric acid, or a combination thereof. In one embodiment, the food acid essentially of citric acid and malic acid.

In one embodiment, the gummy composition further comprises a flavoring agent. In one embodiment, the flavoring agent comprises vanilla, peppermint oil, spearmint oil, eucalyptus oil, cinnamon oil, menthol, monomenthyl succinate, menthol ethylene flycol carbonate, menthone glycerol ketal, menthyl lactate, (-)-isopulegol, p-menthane-3,8-diols, (-)-monomenthyl glutarate, oil of wintergreen (*methylsalicylate*), citrus oils, orange oils, fruit essences, or mixtures or derivatives thereof.

In one embodiment, the gummy composition further comprises a bulking agent. In one embodiment, the bulking agent comprises maltitol syrup, polydextrose, sorbitol, soluble corn fiber, resistant starch, resistant maltodextrin, cellulose, hemicellulose, fructo-oligosaccharides, galacto-oligosaccharides, lactulose, xylo-isomalto-oligosaccharide, soybean oligosaccharide, oligo-glucose, stachyose, lactosucrose, or a combination thereof.

In one embodiment, the flavoring agent comprises of one or more molecular entities that that are less than 500 Da and has part of its or their chemical structure an ester and/or a carboxylic acid and/or an aldehyde and/or an amine and/or an amide and/or a ketone and/or aromatic group and/or vinylic group. In one embodiment, the gummy composition comprises from about 0.01% to about 1% of flavoring agent. In one embodiment, the gummy composition comprises from about 0.01% to about 0.6% of flavoring agent.

In one embodiment, the coloring agent comprises FD&C Blue No. 1, FD&C Blue No. 2, FD&C Green No. 3, FD&C Red No. 3, FD&C Red No. 40, FD&C Yellow No. 5, FD&C Yellow No. 6, Annatto (E160b), Caramel coloring (E150a-d), Carmine (E120), Dactylopius coccus, Elderberry juice (E163), Lycopene (E160d), Paprika (E160c), Turmeric (E100), Spirulina, Titanium Dioxide, Iron Oxide Red, Iron Oxide Black, Iron Oxide Yellow, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), or a combination thereof. In one embodiment, the gummy composition comprises from about 0.01% to about 1% of coloring agent.

In one embodiment, the pH of the composition is about 3 to about 5. In one embodiment, the pH of the composition is about 3.7 to about 4. In one embodiment, the pH is adjusted by the addition of suitable acid, buffer, or both.

In one embodiment, the gummy composition comprises from less than 25% weight of water. In one embodiment, the gummy composition comprises from less than 20% weight of water. In one embodiment, the gummy composition comprises from less than 15% weight of water. In one embodiment, the gummy composition comprises from about 10% to about 15% weight of water.

Gummy compositions in the present disclosure are safer to diabetics to consume due to its low glycemic index. The low GI sugars in the present gummy composition may lead to the composition properties such as, without limitation, low glycemic index, increasing metabolism, helping to maintain joint health, reducing tooth decay and helping to reduce rates of obesity. Some embodiments may have anti-hyperglycemic and anti-hyperlipidemic properties. In one embodiment, the disclosed gummy composition may be used by for obese and diabetic subjects.

In another aspect, the application provides the methods for making the gummy composition disclosed thereof. According to the invention, the method comprises mixing a gelling agent with the active component in hot water to provide a first solution, mixing low GI sugars in hot water to provide a second solution, combining the first solution with the second solution to provide a total mixture, wherein the total mixed is heated to brix 82 to provide a gummy base, and adding food acid into the gummy base to provide a gummy composition.

### BRIEF DESCRIPTION OF THE DRAWINGS

The foregoing and other features of this disclosure will become more fully apparent from the following description and appended claims, taken in conjunction with the accompanying drawings. Understanding that these drawings depict only several embodiments arranged in accordance with the disclosure and are, therefore, not to be considered limiting of its scope, the disclosure will be described with additional specificity and detail through use of the accompanying drawings, in which:
FIGURE 1 shows the Chemical structures for psicose, sorbose, and tagatose and their relation to fructose;
FIGURE 2 shows the glycemic index and calorie per gram of tagatose;
FIGURE 3 shows that trehalose digests and releases glucose very slowly compared to most sugars;
FIGURE 4 shows that isomaltulose digests very slowly and has very low impact on blood glucose levels; and
FIGURE 5 shows the structure of isomalt.

### DETAILED DESCRIPTION

Sugar is a broad term chemically and is often used interchangeably with carbohydrate. The mass of traditional gummy products largely constitutes of "simple sugars," i.e., mono-saccharides or di-saccharides such as glucose, sucrose (table sugar), lactose (milk sugar), or fructose (fruit sugar).

In contrast to simple sugars, complex sugars are carbohydrate molecules having more than 10 sugar monomers. Examples of complex sugars include amylose (a complex sugar found in starches), cellulose (a complex sugar that comprises the structural material of plants), and chitin (a complex sugar that comprises the exoskeletons of many animals).

Sugars come with all kinds of flavors. For example, not all sugars are sweet. Simple sugars such as sucrose and fructose are sweet. Mannose is bitter while raffinose has no taste to it. Lactose has very little sweetness; and fructose can be twice as sweet as table sugar.

The disclosure provides low glycemic index gummy compositions. According to the invention, the gummy composition includes a gelling component in a sufficient amount to provide a cohesive gelled product. In some embodiments, the gummy composition includes a low glycemic index sugar component having a glycemic index of not more than 20. According to the invention, the gummy composition comprises a low glycemic index sugar component having a glycemic index of not more than 35. According to the invention, the gummy composition comprises not less than 50% weight of the low glycemic index sugar component.

In some embodiments, the gummy composition may further include an active component. The active component comprises a vitamin composition, a mineral composition, an amino acid composition, an antioxidant composition, an herbal composition, or an active pharmaceutical ingredient (API) composition.

The gummy composition has a low glycemic index and, therefore, is safe for diabetics to use. In one embodiment, the gummy composition has a GI value of not exceeding 8, 10 or 15. In one embodiment, the gummy composition has a GI value of not exceeding 20 or 25.

The gummy composition comprises low glycemic index (GI) sugars, forming a low glycemic index sugar component. Low GI sugars are simple sugars having a GI value of not exceeding 20. In one embodiment, the gummy composition comprises not less than 60% of low GI sugars.

In some embodiments, the low glycemic index sugar component has a glycemic index of not more than 25, 20, 15, 12, 10, 8, or 5. In some embodiments, the low glycemic index sugar component is not less than 60%, 70%, 75%, 80%, or 90% weight of the gummy composition. According to the invention, the gummy composition comprises not less than 50% weight of the low glycemic index sugar component. In one embodiment, the low glycemic index sugar component is from about 70% to about 75% weight of the gummy composition.

In some embodiments, the low glycemic index sugar component comprises a low glycemic index sugar having a glycemic index of not more than 35 and a molecular weight of not more than 540.

In some embodiments, the low glycemic index sugar component consists essentially of low glycemic index sugars, wherein each low glycemic index sugar has a glycemic index of not more than 35 and a molecular weight of not more than 360.

In some embodiments, the low glycemic index sugar component comprises a low GI monosaccharide, a low GI disaccharide, a low GI tri-saccharide, or a combination thereof. In some embodiments, the low GI monosaccharide comprises a stereoisomer of D-fructose, D-glucose, or D-galactose. In some embodiments, the low GI monosaccharide comprises L-fructose, L-glucose, L-galactose, D- or L-Psicose, D- or L-Sorbose, D- or L-Tagatose, or a combination thereof. In some embodiments, the low GI disaccharide comprises an isomer of D-maltose (1,4- diglucose) or an isomer of D-sucrose (1,2- fructose glucose). In some emboidments, the low GI disaccharide comprises D- or L-trehalose, D- or L-isomaltulose (isomaltuose), or a combination thereof. In some embodiments, the low GI tri-saccharide comprises D- or L-raffinose.

In some embodiments, the low GI sugar component comprises D-trehalose and D-isomaltulose. It is a surprising finding that D-trehalose and D-isomaltulose combination has synergistically enhanced sweetness taste profile, which is similar to the natural and clean sweetness profile of sucrose. In some embodiments, the low GI sugar component consists essentially of D-trehalose, D-isomaltulose and a third low GI sugar. The third low GI sugar may be L-fructose, L-glucose, L-galactose, D-psicose, D- or L-sorbose, D- or L-tagatose, or a combination thereof. In some embodiments, the low GI sugar component comprises D-trehalose, D-isomaltulose, and D-psicose. In some embodiments, the low GI sugar component comprises D-isomaltulose, D-psicose, and D or L-tagatose. In some embodiments, the low GI sugar component comprises D-isomaltulose and D-psicose. In some embodiments, the low GI sugar component comprises D-trehalose and D-psicose. According to the invention, the low glycemic index sugar component comprises trehalose, isomaltulose and psicose.

In some embodiments, the gummy composition may be substantially free of non-sugar sweeteners, sugar substitutes, or sugar alcohols. In some embodiments, the gummy composition may be substantially free of high glycemic sugars including, without limitation, D-sucrose, D-fructose, D-maltose, or D-glucose. According to the invention, the gummy composition is substantially free of D-glucose, D-fructose, D-sucrose, and sugar alcohols. In some embodiments, the gummy composition has a sweetness taste profile similar to D-sucrose.

In one embodiment, the gummy compositions may increase metabolism and promote weight loss. In one embodiment, the gummy compositions, unlike traditional "sugar free" (sugar alcohol) products, may prevent or be void of gastric distress. In one embodiment, the gummy compositions have a sweetness profile substantially similar to table sugar (sucrose). In some embodiments, the gummy compositions have a sweet and good tasting like a "conventional" sugar gummy candy, but with the health benefits of prebiotics and lack of negatives that "conventional" sugar gummy candy have, such as high glycemic index.

In one embodiment, the gummy composition comprises a gelling agent, a low GI sugar component and an organic acid. In one embodiment, the gummy composition further comprises flavoring agent. In one embodiment, the gummy composition further comprises coloring agent. In one embodiment, the gummy composition further comprises an active component.

### Gelling Component

A large part of the texture of gummy products comes from the gelling agents used. The gelling agents are typically high molecular weight polymers.

Pectin is a heteropolysaccharide consisting of galactogluconic acid found in plant cell walls that gives plants their rigid structure. In some embodiment, pectin gels in the presence of sugar and acid. In some embodiment, chemically modified pectin may gel in the presence of calcium or potassium ions instead of acid, which makes the pectin to be useful in low-acid compositions. Pectin is watersoluble. It's easier to dissolve in water when mixed with sugar. Pectin may be used in combination with gelatin. Gelatin provides a tough chewiness, while pectin lends a distinct tenderness.

Gelatin is a protein made from animal collagen. The source of collagen is often derived from cattle and pigs, but sometimes fish and chicken. Gelatin may be combined with other hydrocolloids-pectin, agar, starch, gum Arabic-to create unique mouth-feel textures.

Starch is the major energy storage macromolecule found in plants such as wheat, corn, potatoes, and tapioca. There are two kinds of starch molecules: amylose and amylopectin, which are both based upon the alpha form of glucose. Amylose starches gel when heated; amylopectin starches don't. For gummy applications the starch is often "modified". There are a variety of modification techniques including for example contacting starch with acid, sodium or potassium hydroxide, or oxidizing the starch. These treatments help the starch to dissolve in water and gel to an appropriate level.

Agar or agar-agar is a jelly-like substance, obtained from algae. Agar is derived from the polysaccharide agarose. Agar may be the mixture of two components: the linear polysaccharide agarose, and a heterogeneous mixture of smaller molecules called agaropectin. Agar gels tend to weep or extrude water over time when used by itself as a gelling agent. Thus, agar gels often include locust bean gum. Locust bean gum helps to prevent weeping of agar gels. Locust bean gum is a galactomannan polysaccharide vegetable gum extracted from the seeds of the carob tree. The two polysaccharides from agar and locust bean gum synergistically interact with each other to form a strong gel that does not weep.

Carrageenans or carrageenins are a family of linear sulfated polysaccharides that are extracted from red edible seaweeds. The linear saccharide chains have a tendency to curl to form helical structures. Kappa-carrageenan has one sulphate group per disaccharide and forms strong, rigid gels in the presence of potassium ions. Similar agar, locust bean gum is often used with kappa-carrageenan to prevent water from being expelled from the bulk of the gel (weeping). Gels formed from kappa-carrageenan and potassium ions are thermally reversible. That is they melt when heated and solidify when cooled.

Alginic acid is a linear copolymer with homopolymeric blocks of (1-4)-linked β-D-mannuronate (M) (acid form of mannose) and its C-5 epimer α-L-guluronate (G) (acid form of gulose) residues, respectively, covalently linked together in different sequences or blocks. The monomers can appear in homopolymeric blocks of consecutive G-residues (G-blocks), consecutive M-residues (M-blocks) or alternating M and G-residues (MG-blocks). Alginate forms strong hydrogels when cross-linked with calcium ions.

### Low GI sugar component

In some embodiments, low GI sugars disclosed herein may help to bind the gummy composition together through interaction with the gelling agent. In some embodiments, the low GI sugars help to keep the texture of the composition in a soft gum like state by acting as a humectant. The low GI sugars bind water. By binding water, the low GI sugars prevent the composition from crystalizing or drying out, therefore giving the composition a chewy texture. In some embodiments, the low GI sugars provide a nutritional aspect to the gummy products.

### Monosaccharides

In some embodiments, the low GI sugar component comprises monosaccharides. In some embodiment, the low GI sugar may be isomers of D-glucose or D-fructose.

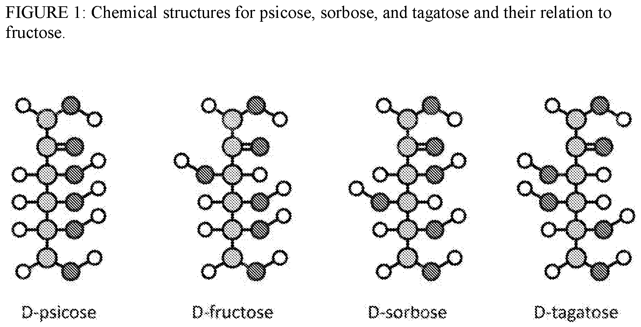

Psicose, sorbose, and tagatose are mono-saccharides and are isomers of D-fructose. They are ketoses and are C3-C5 stereoisomers of fructose. Psicose is identical to sucrose (table sugar) in sweetness but has nearly zero calories and does not promote tooth decay. Tagatose is nearly as sweet as sucrose yet only has 38% of the caloric value of sucrose and is much more tooth friendly than sucrose. Sorbose is equivalent to sucrose in sweetness. Since these are sugars (carbohydrates) like fructose, glucose, and sucrose, they cook and behave like sugars without the caloric significance of sucrose. Psicose has 70% the sweetness of sucrose while tagatose and sorbose are nearly as sweet as table sugar. These are natural sugars that are fully absorbable by the body, but they are very low in calorie and have minimal impacts on blood sugar levels. They are excellent humectants and prevent the gummy composition disclosed herein from drying out. These monosaccharides have strong antihyperglycemic and antihyperlipidemic effects. The chemical structures for psicose, sorbose, and tagatose are shown in FIGURE 1.

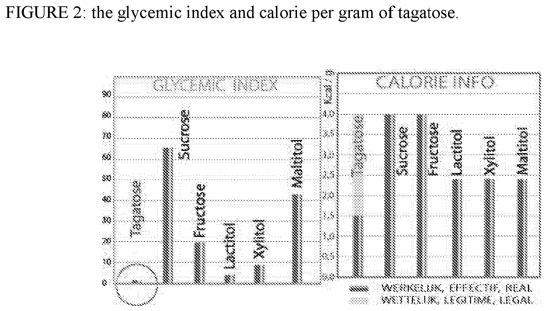

Psicose, sorbose, and tagatose are much lower in glycemic index than glucose or sucrose levels. The low glycemic index and low-calorie count is shown in FIGURE 2. The properties of psicose, sorbose and tagatose make them suitable for type I and II diabetics while also promoting reduction in obesity.

### Disaccahrides

In some embodiments, the low GI sugar component comprises di-saccharides. The monomer unit in the di-saccharides may be hexose (such as glucose) or pentose (such as fructose). In some embodiments, the disaccharide comprises a glucose unit, a fructose unit, or a combination thereof. In some embodiments, the disaccharide may have two glucose units linked together with a glycosidic bond other than alpha-1,6-glycosidic bond, such as 1,1-glucoside bond, 1,2-glucoside bond, 1,3-glucoside bond, 1,4-glucoside bond, 1,5-glucoside bond, or a beta-glycosidic bond. In some embodiments, the disaccharide comprises a glucose unit and a fructose unit linked together with a glycosidic bond other than alpha-1,4-glycosidic bond, such as 1,1-glycosidic bond, 1,2-glycosidic bond, 1,3-glycosidic bond, 1,5-glycosidic bond, or a beta-glycosidic bond.

Trehalose, also known as mycose or tremalose, is a natural alpha-linked disaccharide formed by an α,α-1,1-glucoside bond between two α-glucose units. Trehalose is a non-reducing sugar and does not contain nucleophilic groups in its molecule. However, trehalose is reported to have antioxidant effects and neurological benefits.

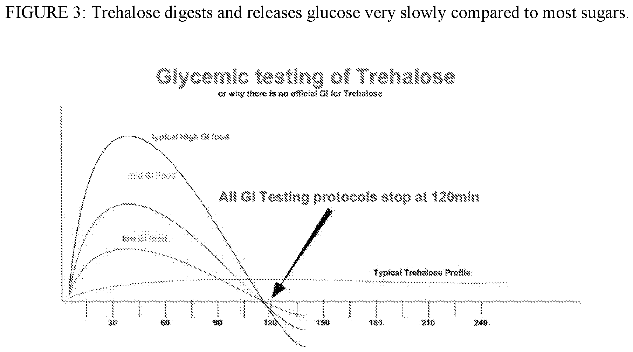

Trehalose is digested in the small intestine by the enzyme trehalase to release two molecules of glucose. As shown in FIGURE 3, trehalose does not cause a spike in glucose levels. Rather, trehalose leads to a long-sustained release of glucose at a low level, meaning the glucose levels slowly rise and are sustained over a longer period of time. The sustained release of glucose prevents wild swings in insulin levels that other sugars cause. Furthermore, there is no depletion of glucose over the long term, which is very unlike high glycemic index sugars. This is due to the digestion of trehalose in the small intestine rather than the mouth. Trehalose has a much lower instance of causing dental decay than most carbohydrates, which is another benefit of the technology.

Isomaltulose (palatinose^{®}) is a derivative of a natural source of sucrose. Isomaltulose is made by enzymatic rearrangement of the alpha-1,2 bond between the glucose and the fructose molecule to an alpha-1,6 bond. Isomaltulose is digested by enzymatic action by the enzyme sucrase. However, due to the rearrangement of palantinose vs. sucrose, sucrase hydrolysis of isomaltulose is much slower. Like trehalose, isomaltulose only has a minor influence on the blood glucose level and a low glycemis index. As shown in FIGURE 3, isomaltulose has a low glycemic index (about 30) and therefore helps to stabilize the insulin level in the body. The products of the hydrolysis are glucose and fructose. The glucose can then be used by the body for energy while the fructose is converted to glucose by the liver. Due to its slow and full absorption and metabolic hydrolysis, isomaltulose release glucose slowly and supplies constant and long-lasting energy to the body. Isomaltulose also has lower instances of tooth decay.

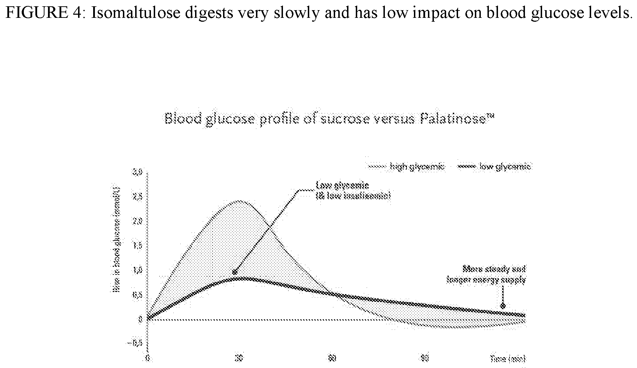

As shown in FIGURE 4, isomaltulose does not cause a spike in glucose levels. While the caloric density of isomaltulose is 4 Cal/g like other sugars, the lack of blood sugar level spikes makes isomaltulose safer for diabetics.

During the process of experimenting the low GI gummy composition, the applicant unexpectedly discovered that the mixture of trehalose and isomaltulose is synergistically sweeter than each individual component through a group panel testing. The combination of the two sugars provides synergistically enhanced sweetness taste while retaining the natural sweet profile similar to sucrose (conventional sugar taste). In one embodiment, the low GI sugar component comprises maltitol and isomalt. In one embodiment, the low GI sugar component comprises maltitol and allulose. In one embodiment, the low GI sugar component comprises maltitol and tagatose. In one embodiment, the low GI sugar component comprises isomalt and allulose. In one embodiment, the low GI sugar component comprises isomalt and tagatose. In one embodiment, the low GI sugar component comprises maltitol, isomalt and allulose. In one embodiment, the low GI sugar component comprises maltitol, isomalt and tagatose,

Gummy confectioneries are concentrated carbohydrate-water solutions where the carbohydrates can range 50 - 85% of the gummy mass and the water 10 - 25% of the gummy mass. A challenge is to prevent crystallization of the carbohydrates to maintain a smooth gummy texture and the stability of the product over its shelf life. Water solubility of the carbohydrate is an important feature. However, even highly soluble carbohydrates can crystalize when highly concentrated. The application disclosed methods and composition for preventing crystallization of a low GI gummy composition.

Carbohydrates have a specific kind of stereochemistry to them called epimers since there are often more than one stereo center. Examples of carbohydrates with the same connectivity but are mirror images of each other at specific stereo centers are glucose, mannose, galactose for some aldohexoses or fructose, psicose, tagatose, sorbose for some ketohexoses. Th applicant discovered that mixing epimers can prevent the component from crystallizing. The mirror image structure is "out of place" which prevents lattice formation.

The application provides compositions and methods for a stable low GI gummy composition.

### Other sweetening agents

N-acetyl glucosamine is a naturally sweet carbohydrate. N-acetyl glucosamine is essential for optimal health and function in the body. In some embodiment, the low glycemic index gummy composition comprises N-acetyl glucosamine.

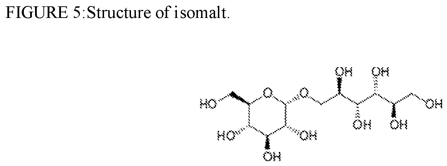

Isomalt is a sugar-alcohol but is different from sorbitol or xylitol in that it is a dimer between glucose and sorbitol or glucose and mannitol. The structure of isomalt is shown in FIGURE 5. Isomalt does not promote tooth decay and has half the calories of normal sugar. Isomalt has similar sweetness profile as sugar and largely bypasses the metallic taste of sorbitol and xylitol. It cooks like normal table sugar.

However, the body lacks the enzyme to hydrolyze isomalt. Stomach acid does hydrolyze some isomalt to yield a glucose molecule and either a sorbitol or mannitol molecule (which provides some calories). However, most of the isomalt does not hydrolyze and travels through the digestive track where, like other sugar alcohols, it can cause gastric stress. In some embodiments, the gummy composition may include isomalt.

Muramic Acid is a sugar-acid and an amino sugar. It is the ether derivative of N-glucosamine and lactic acid. Muramic Acid may improve joint health as well as help enhance collagen production. The N-acetylmuramic acid is the common form found in nature. It is found in Spirulina. In some embodiments, the gummy composition may include muramic acid or N-acetylmuramic acid.

### Vitamins

The vitamin composition may include vitamins or their precursors thereof. In some embodiments, the composition includes a vitamin B composition. In some embodiments, the Vitamin B composition consists of folate, B6, and B 12. In some embodiments, the vitamin B composition consists of thiamine (B1), riboflavin (B2), niacin (B3), panotothenic acid (B5), pyridoxine (B6), biotin (B7), folic acid (B9), cobalamins (B12), and derivatives or combinations thereof. In some embodiments, per daily dosing, the composition comprises at least 300mcg, 400mcg, 450mcg, or 500mcg of folate, at least 3mg, 4.5mg, 5.5mg, 6.5mg of B1, at least 3mg, 5mg, 6mg, or 7mg of B2, at least 12mg, 15mg, 16mg, 18mg, or 20mg of niacinamide, at least 4mg, 6mg, 7mg, 8mg, 10mg of B6, at least 15mcg, 20mcg, 30mcg, 50mcg, 1mg, 2mg, 4mg, 5mg, or 6mg of B12, at least 30mcg, 40mcg, 50mcg, 80mcg, or 1mg of biotin, or at least 7mg, 10mg, 12mg, 15mg, or 20mg of pantothenic acid.

In some embodiments, vitamins can include fat-soluble vitamins such as vitamin A, vitamin D, vitamin E, and vitamin K and combinations thereof. In some embodiments, vitamins can include water-soluble vitamins such as vitamin C (ascorbic acid), the B vitamins, and choline.

### Minerals

The mineral composition may include ions of sodium, magnesium, chromium, iodine, iron, manganese, calcium, copper, fluoride, potassium, phosphorous, molybdenum, selenium, zinc, and combinations thereof. The minerals may be in the forms of salts or chelates.

In some embodiments, per daily dosage, the composition includes from at least 800mg, 1000mg, 1200mg, or 1500mg of calcium. In some embodiment, per daily dosage, the composition includes at least 10mg, 15mg, 20mg, or 30mg of iron.

### Amino acid

The amino acid composition may include natural or non-natural amino acids. In some embodiments, the amino acid composition includes leucine, isoleucine, valine, arginine, glutamine, alanine, carnitine, creatine, tryptophan, aspartate, ornithine, tyrosine, taurine, lysine, histidine, threonine, phenylalanine, methionine, or any derivative thereof. In some embodiments, the amino acid composition comprises leucine, isoleucine, valine and a combination thereof. In some embodiments, the amino acid composition comprises leucine, isoleucine, and valine at a ratio of 2:1:1.

In some embodiments, per daily dosage, the gummy composition includes at least 100mg, 150mg, 200mg, or 300mg of the amino acid composition.

### Antioxidants

In some embodiments, the antioxidant composition can include but are not limited to ascorbic acid, citric acid, rosemary oil, vitamin A, vitamin E, vitamin E phosphate, tocopherols, di-alpha-tocopheryl phosphate, tocotrienols, coenzyme Q10, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof. In some embodiments, the antioxidant composition may include extracts and active phytochemicals such as ferulic acid (from apples), ginseng, ginko biloba, beta carotene, capsicanoids, anthocyanidins, bioflavinoids, d-limonene, isothiocyanates, cysteines from garlic, ginger, grapes, catechins and polyphenols from teas, onions, phytosterols, isoflavones, lycopene, curcumin, caffeine, glucosamine, chondroitin, msm, melatonin, serotonin, and mixtures thereof.

In some embodiments, the antioxidant composition consists essentially of vitamin E, beta-carotene, and vitamin C.

### Herbal composition

The composition may include an herbal composition including herbal extracts, isolates or active phytochemicals. Examples herbal extracts and isolates include without limitation ginseng, ginko biloba, echinacea, spirulina, garlic, ginger, Leucojum aestivum, Ganoderma Lucidum, grapes, thermadrene, ma-huang, guarana, caffeine, purple willow bark, cayenne pepper, Kava kava (*kava*), St. Johns wort, Gamma oryzanol and *Tribulus terrestris, Cordyceps sinensis, Rhodiola rosea,* and *Cytoseira canariensis.* Example phytochemicals may include ferulic acid beta carotene, capsicanoids, anthocyanidins, bioflavinoids, d-limonene, isothiocyanates, cysteines, catechins and polyphenols, onions, phytosterols, isoflavones, lycopene, curcumin, caffeine, inositol hexanicotinate, glucosamine, chondroitin, msm, melatonin, serotonin, cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate (EGCG), theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, or combinations thereof.

Ginseng consists of several species belonging to the plant family Araliaceae. The two major forms are Chinese, Korean or Asian ginseng that belong to the genus *Panax,* and Siberian or Russian ginseng, which belongs to the genus *Eleutherococcus.* The biologically active constituents in *Panax ginseng* are a complex mixture of triterpene saponins known as ginsenosides. Siberian, or Russian, ginseng consists of the dried roots and rhizome of *Eleutherococcus senticosis,* and contains phenolics, polysaccharides, and eleutherosides. In China, *Eleutherococcus senticosus* is known as *wujiaseng* or *Ciwujia* with active ingredients as ciwujianosides.

In some embodiments, the herbal composition comprises ginseng, schisandra, dang-gui, he-shou-wu, gotu kola (*centella asiatica*), ashwagandha, *tribulus terrestris, mucunna pruriens,* ciwujia, or extracts or isolates thereof. In some embodiments, the herbal composition comprises ginseng or its extract or isolate thereof. In some embodiments, the ginseng comprises Asian ginseng (panax ginseng), Siberian ginseng (Eleutherococcus senticosus), American ginseng (Panax quinquefolius), indian ginseng (Withania somnifera), or a combination thereof. In some embodiments, the herbal composition comprises essentially ginseng, or its extract or isolate thereof. In some embodiments, the herbal composition comprises at least 100mg, 200mg, 300mg, 500mg, 1g of ginseng extract or isolate thereof.

In some embodiments, the herbal composition consists of ginseng and Ganoderma Lucidum. In some embodiments, the herbal composition consists of ginseng and dang-gui, or its extract or isolate thereof. In some embodiments, the herbal composition consists of ginseng and ciwujia, or its extract or isolate thereof. In some embodiments, the herbal composition consists of ginseng and ma-huang, or its extract or isolate thereof.

In some embodiments, the herbal composition comprises Astragalus powder, extract, isolates, ingredients, or its derivatives. Example Astragalus active ingredients may include without limitation calycosin-7-O-beta-D-glucodise, Astragaloside I, II IV, calycosin, formononetin, isoquercitrin, Ononin, polysaccahrides, flavonoids, or a derivative thereof. In some embodiments, the herbal composition comprises Astragalus and dang-gui. In some emboidmetns, the herbal composition comprises Asrtagalus and ginseng.

In some embodiments, per daily dosage, the gummy composition includes at least 10mg, 20mg, 50mg, 100mg, 200mg, 300mg, 500mg, 800mg, 1000mg or 2000mg of the herb composition. The herbal composition may be any amount in between the ranges.

### Fibers, prebiotics and probiotics

The composition may further include fibers or prebiotics. In one embodiment, the fibers may present in an amount of from about 0.001% to 80%, alternatively 0.001% to 5%, alternatively 5% to 10%, alternatively 10% to 15%, alternatively 15% to 40%, alternatively 40% to 60%, alternatively 60% to 80%, by weight of said composition. Any suitable fiber can be used. In one embodiment, a naturally derived fiber is used, including one or more selected from naturally derived inulin, inulin extract, synthetic inulin, hydrolysis products of inulin commonly known as fructooligosaccharides, galacto-oligosaccharides, xylooligosaccharides, oligo derivatives of starch, husks, brans, psyllium, polysaccharides, starches, polycarbophil, lignin, arabinogalactans, chitosans, oat fiber, soluble corn fiber, non-digestible corn dextrin, non-digestible wheat dextrin, locust bean gum and derivatives of locust bean gum, hydroxypropylmethyl cellulose (HPMC), pectin, and mixtures thereof.

In some embodiments, fibers may include inulin, wheat dextrin, or fructooligiosaccharides. Inulin, wheat dextrin, and fructooligiosaccharides may also act as a thickening agent and improve the texture of the composition. Various load rates of dietary fiber can be incorporated in the composition to create improved texture and at certain load rates can provide dietary benefits including promoting a healthy digestion system, controlling blood sugar levels, and providing probiotic benefits. The addition of the dietary fiber along with the remaining components allow for the addition of water that helps displace sugar within the flavored chewy or gummy confection.

Inulin is a highly effective prebiotic, stimulating the growth of beneficial probiotic bacteria in the gut. Inulin is used in low fat products because of its ability to provide a creamy smooth texture to products. Inulin is a dietary fiber and is believed to activate beneficial good bacteria in the digestive tract. The activation of these bacteria is thought to reduce the risk of bowel cancer. Inulin has a mildly sweet taste, but does not affect blood sugar levels and is recommended for diabetics. Inulin has been clinically proven to increase calcium absorption. In some embodiments, the application provides gummy composition with inulin as the fiber component and calcium as the mineral component for calcium supplementation. This gummy composition provides an improved absorption of calcium because of the inulin within its composition.

In some embodiments, the composition may further include probiotics. Example probiotics include, but not limited to, lactic acid producing microorganisms such as Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarum, Lactobacillus jenseni, Lactobacillus casei, Lactobacillus fermentum, Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus and mixtures thereof. Breath fresheners are also known by the following trade names: Retsyn^{™}, Actizol^{™}, and Nutrazin^{™}. Examples of malodor-controlling compositions are also included in U.S. Pat. No. 5,300,305 to Stapler et al. and in U.S. Patent Application Publication Nos. 2003/0215417 and 2004/0081713 .

### Sweetener

In some embodiments, a limited amount of sweetener may be used. In some embodiments the amount of sweetener may be present in amounts from about 0.001% to about 3%, by weight of the composition, depending upon the sweetener or combination of sweeteners used. The exact range of amounts for each type of sweetener may be selected by those skilled in the art.

Sweeteners may include one or more monosaccharides or disaccharides. Examples include sugar, sucrose, invert sugar, dextrose, lactose, honey, malt syrup, malt syrup solids, maltose, fructose, granular fructose, maple syrup, rice syrup, rice syrup solids, sorghum syrup, refiners syrup, corn syrup, corn syrup solids, high fructose corn syrup, molasses, or combinations thereof.

In one embodiment, the sweetener include common sugars such as sucrose and glucose, polyols such as maltitol, erythritol, and isomalt, syrup sweeteners such as glucose syrup, corn syrup, high fructose corn syrup, and juice concentrates.

In one embodiment, artificial sweeteners can be used such as acesulfame K, aspartame, sucralose, d-tagatose, neotame, monatin, and acesulfame potassium (Ace-K), or combinations thereof. In one embodiment, the sweetener may be extract, isolate, or derivatives from stevia, monk fruit, katemfe fruit, or licorice.

The sweeteners involved may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:
(a) water-soluble sweetening agents such as dihydrochalcones, monellin, steviosides, lo han quo (monk fruit), lo han quo derivatives, glycyrrhizin, dihydroflavenol, thaumatin, and sugar alcohols such as sorbitol, mannitol, maltitol, xylitol, erythritol, and L-aminodicarboxylic acid aminoalkenoic acid ester amides, such as those disclosed in U.S. Pat. No. 4,619,834,
   and mixtures thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
(c) dipeptide based sweeteners, such as L-aspartic acid derived sweeteners, such as L-aspartyl-L-phenylalanine methyl ester (Aspartame), N--[N-(3,3-dimethylbutyl)-L-.alpha.-aspartyl]-L-phenylalanine 1-methyl ester (Neotame), and materials described in U.S. Pat. No. 3,492,131, L-alphaaspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, and mixtures thereof;
(d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-- furanoside, or 4,1'-dichloro-4,1'-dideoxygalactosucrose; 1',6'-dichloro 1',6'-dideoxysucrose; 4-chloro-4-deoxy-alpha-D-galactopyranosyl-1,6-dichloro-1,6-dideoxy-beta-D- - fructofuranoside, or 4,1',6'-trichloro-4,1',6'-trideoxygalactosucrose; 4,6-dichloro-4,6-dideoxy-alpha-D-galactopyranosyl-6-chloro-6-deoxy-beta-D- -fructofuranoside, or 4,6,6'-trichloro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideo- xy-beta-D-fructofuranoside, or 4,6,1',6'-tetrachloro-4,6, 1',6'-tetradeoxygalacto-sucrose; and 4,6,1',6'-tetradeoxy-sucrose, and mixtures thereof;
(e) protein-based sweeteners such as thaumaoccous danielli (Thaumatin I and II) and talin; and
(f) the sweetener monatin (2-hydroxy-2-(indol-3-ylmethyl)-4-aminoglutaric acid) and its derivatives.

The intense sweetening agents may be used in many distinct physical forms well known in the art to provide an initial burst of sweetness and/or a prolonged sensation of sweetness. Without being limited thereto, such physical forms include free forms, spray dried forms, powdered forms, beaded forms, encapsulated forms, and mixtures thereof. In one embodiment, the sweetener is a high intensity sweetener such as aspartame, sucralose, and acesulfame potassium (e.g., Ace-K or acesulfame-K).

In some embodiments, the sweetener may be a polyol. Polyols can include, but are not limited to glycerol, sorbitol, maltitol, maltitol syrup, mannitol, isomalt, erythritol, xylitol, hydrogenated starch hydrolysates, polyglycitol syrups, polyglycitol powders, lactitol, and combinations thereof.

In general, an effective amount of intense sweetener may be utilized to provide the level of sweetness desired, and this amount may vary with the sweetener selected. The intense sweetener may be present in amounts from about 0.001% to about 3%, by weight of the composition, depending upon the sweetener or combination of sweeteners used. The exact range of amounts for each type of sweetener may be selected by those skilled in the art.

In one embodiment, the gummy composition comprises allulose and monk fruit extract. In one embodiment, the gummy composition comprises allulose, tagatose and monk fruit extract.

### Food acids

The pH of the composition is about 3 to about 5, about 3.7 to about 4. The pH may be adjusted by a food acid, buffer, or both. Suitable food acids include but are not limited to acetic acid, adipic acid, ascorbic acid, butyric acid, citric acid, formic acid, fumaric acid, glyconic acid, lactic acid, phosphoric acid, malic acid, oxalic acid, succinic acid, tartaric acid, or combinations thereof.

Suitable buffers include but are not limited to sodium citrate and potassium citrate. For example, an acid/buffer system is 1.33% of a 54% citric acid solution, buffered with sodium citrate.

The amount of acid will be in the typical range of from about 0.5 to about 2% by weight, e.g., about 1.25%. Higher acid (lower pH) results in a lack of structure while lower acid levels do not provide enough "acid bite" in the flavor profile.

### Flavoring agents

In some embodiments, the composition may further include a flavoring agent. Flavoring agents may include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof.

In some embodiments, the flavoring agents may include mint(s), menthol, menthone, isomenthone, camphor and eucalyptol, eucalyptol, camphor, borneol, fenchone, menthone and isomenthone, isopulegol, monomenthyl succinate, and menthyl lactate, menthone, isomenthone, borneol, fenchone, eucalyptus, ducalyptol, ethyl benzoate, neomenthol, d-fenchone, furfurylidene butyrate, bucchu fractions, sage oil, corn mint oil, rosemary, monomenthyl succinate, amyl salicylate, eugenol, phellendrene, propyl furoate, ethyl-3-hydroxy butyrate, hexyl valerate, anisyl propionate, anysyl butyrate, dihydrocarveol, or clary sag,

Non limiting representative flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, apricot, banana, melon, apricot, ume, cherry, raspberry, blackberry, tropical fruit, mango, mangosteen, pomegranate, papaya and so forth. Other potential flavors whose release profiles can be managed include a milk flavor, a butter flavor, a cheese flavor, a cream flavor, and a yogurt flavor; a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with the cooling agents, described herein below. In some embodiments, flavorants may choose from geraniol, linalool, nerol, nerolidal, citronellol, heliotropine, methyl cyclopentelone, ethyl vanillin, maltol, ethyl maltol, furaneol, alliaceous compounds, rose type compounds such as phenethanol, phenylacetic acid, nerol, linalyl esters, jasmine, sandlewood, patchouli, and/or cedarwood.

In some embodiments, other flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used. These may include natural as well as synthetic flavors.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, e.g., melonal (melon), 2,6-dimethyloctanal (green fruit), and 2-dodecenal (citrus, mandarin), cherry, grape, blueberry, blackberry, strawberry shortcake, and mixtures thereof.

In some embodiments, flavoring agents are used at levels that provide a perceptible sensory experience i.e. at or above their threshold levels. In other embodiments, flavoring agents are used at levels below their threshold levels such that they do not provide an independent perceptible sensory experience. At subthreshold levels, the flavoring agents may provide an ancillary benefit such as flavor enhancement or potentiation.

In some embodiments, a flavoring agent may be employed in either liquid form and/or dried form. When employed in the latter form, suitable drying means such as spray drying the liquid may be used. Alternatively, the flavoring agent may be absorbed onto water soluble materials, such as cellulose, starch, sugar, maltodextrin, gum arabic and so forth or may be encapsulated. In still other embodiments, the flavoring agent may be adsorbed onto silicas, zeolites, and the like.

In some embodiments, the flavoring agents may be used in many distinct physical forms. Without being limited thereto, such physical forms include free forms, such as spray dried, powdered, beaded forms, encapsulated forms, and mixtures thereof.

### Sensate agents

In some embodiment, the composition further includes a sensate agent. Sensate agents can include cooling agents, warming agents, tingling agents, effervescent agents, and combinations thereof. A variety of cooling agents may be employed. For example, among the useful cooling agents are included xylitol, erythritol, dextrose, sorbitol, menthane, menthone, ketals, menthone ketals, menthone glycerol ketals, substituted p-menthanes, acyclic carboxamides, mono menthyl glutarate, substituted cyclohexanamides, substituted cyclohexane carboxamides, substituted ureas and sulfonamides, substituted menthanols, hydroxymethyl and hydroxymethyl derivatives of p-menthane, 2-mercapto-cyclo-decanone, hydroxycarboxylic acids with 2-6 carbon atoms, cyclohexanamides, menthyl acetate, menthyl salicylate, N,2,3-trimethyl-2-isopropyl butanamide (WS-23), N-ethyl-p-menthane-3-carboxamide (WS-3), isopulegol, 3-(1-menthoxy)propane-1,2-diol, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, 3-(1-menthoxy)ethan-1-ol, 3-(1-menthoxy)propan-1-ol, 3-(1-menthoxy)butan-1-ol, 1-menthylacetic acid N-ethylamide, 1-menthyl-4-hydroxypentanoate, 1-menthyl-3-hydroxybutyrate, N,2,3-trimethyl-2-(1-methylethyl)-butanamide, n-ethyl-t-2-c-6 nonadienamide, N,N-dimethyl menthyl succinamide, substituted p-menthanes, substituted p-menthane-carboxamides, 2-isopropanyl-5-methylcyclohexanol (from Hisamitsu Pharmaceuticals, hereinafter "isopregol"); menthone glycerol ketals (FEMA 3807, tradename FRESCOLAT.RTM. type MGA); 3-1-menthoxypropane-1,2-diol (from Takasago, FEMA 3784); and menthyl lactate; (from Haarman & Reimer, FEMA 3748, tradename FRESCOLAT.RTM. type ML), WS-30, WS-14, Eucalyptus extract (p-Mehtha-3,8-Diol), Menthol (its natural or synthetic derivatives), Menthol PG carbonate, Menthol EG carbonate, Menthol glyceryl ether, N-tertbutyl-p-menthane-3-carboxamide, P-menthane-3-carboxylic acid glycerol ester, Methyl-2-isopryl-bicyclo (2.2.1), Heptane-2-carboxamide; and Menthol methyl ether, and menthyl pyrrolidone carboxylate among others. These and other suitable cooling agents are further described in the following U.S. patents: U.S. Pat. Nos. 4,230,688; 4,032,661; 4,459,425; 4,136,163; 5,266,592; 6,627,233.

In some embodiments, warming components may be selected from a wide variety of compounds known to provide the sensory signal of warming to the user. These compounds offer the perceived sensation of warmth, particularly in the oral cavity, and often enhance the perception of flavors, sweeteners and other organoleptic components. In some embodiments, useful warming compounds can include vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethylether, gingerol, shogaol, paradol, zingerone, capsaicin, dihydrocapsaicin, nordihydrocapsaicin, homocapsaicin, homodihydrocapsaicin, ethanol, isopropyl alcohol, isoamylalcohol, benzyl alcohol, glycerine, and combinations thereof.

In some embodiments, a tingling sensation can be provided. One such tingling sensation is provided by adding jambu, oleoresin, or spilanthol to some examples. In some embodiments, alkylamides extracted from materials such as jambu or sanshool can be included. Additionally, in some embodiments, a sensation is created due to effervescence. Such effervescence is created by combining an alkaline material with an acidic material. In some embodiments, an alkaline material can include alkali metal carbonates, alkali metal bicarbonates, alkaline earth metal carbonates, alkaline earth metal bicarbonates and mixtures thereof. In some embodiments, an acidic material can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Examples of "tingling" type sensates can be found in U.S. Pat. No. 6,780,443.

Sensate agents may also be referred to as "trigeminal stimulants" such as those disclosed in U.S. Patent Application No. 205/0202118. Trigeminal stimulants are defined as an orally consumed product or agent that stimulates the trigeminal nerve. Examples of cooling agents which are trigeminal stimulants include menthol, WS-3, N-substituted p-menthane carboxamide, acyclic carboxamides including WS-23, methyl succinate, menthone glycerol ketals, bulk sweeteners such as xylitol, erythritol, dextrose, and sorbitol, and combinations thereof. Trigeminal stimulants can also include flavors, tingling agents, Jambu extract, vanillyl alkyl ethers, such as vanillyl n-butyl ether, spilanthol, Echinacea extract, Northern Prickly Ash extract, capsaicin, capsicum oleoresin, red pepper oleoresin, black pepper oleoresin, piperine, ginger oleoresin, gingerol, shoagol, cinnamon oleoresin, cassia oleoresin, cinnamic aldehyde, eugenol, cyclic acetal of vanillin and menthol glycerin ether, unsaturated amides, and combinations thereof.

In some embodiments, sensate agents are used at levels that provide a perceptible sensory experience i.e. at or above their threshold levels. In other embodiments, sensate components are used at levels below their threshold levels such that they do not provide an independent perceptible sensory experience. At subthreshold levels, the sensates may provide an ancillary benefit such as flavor or sweetness enhancement or potentiation.

### Freshening agents

In some embodiments, the composition further includes a freshening agent. Freshening agents may include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments freshening agent can include but are not limited to zinc citrate, zinc acetate, zinc fluoride, zinc ammonium sulfate, zinc bromide, zinc iodide, zinc chloride, zinc nitrate, zinc fluorosilicate, zinc gluconate, zinc tartarate, zinc succinate, zinc formate, zinc chromate, zinc phenol sulfonate, zinc dithionate, zinc sulfate, silver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof.

### Coloring agents

Coloring agents may be used in amounts effective to produce the desired color. The coloring agents may include pigments which may be incorporated in amounts up to about 6%, by weight of the composition. For example, titanium dioxide may be incorporated in amounts up to about 2%, and preferably less than about 1%, by weight of the composition. The colorants may also include natural food colors and dyes suitable for food, drug and cosmetic applications. These colorants are known as F.D.& C. dyes and lakes. The materials acceptable for the foregoing uses are preferably water-soluble. Illustrative nonlimiting examples include the indigoid dye known as F.D.& C. Blue No. 2, which is the disodium salt of 5,5-indigotindisulfonic acid. Similarly, the dye known as F.D.& C. Green No. 1 comprises a triphenylmethane dye and is the monosodium salt of 4-[4-(N-ethyl-p-sulfoniumbenzylamino) diphenylmethylene]-[1-(N-ethyl-N-p-sulfoniumbenzyl)-delta-2,5-cyclohexadi- eneimine]. A full recitation of all F.D.& C. colorants and their corresponding chemical structures may be found in the Kirk-Othmer Encyclopedia of Chemical Technology, 3rd Edition, in volume 5 at pages 857- 884.

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E160b), bixin, norbixin, astaxanthin, dehydrated beets (beet powder), beetroot red/betanin (E162), ultramarine blue, canthaxanthin (E161g), cryptoxanthin (E161c), rubixanthin (E161d), violanxanthin (E161e), rhodoxanthin (E161f), caramel (E150(a-d)), .beta.-apo-8'-carotenal (E160e), beta.-carotene (E160a), alpha carotene, gamma carotene, ethyl ester of beta-apo-8 carotenal (E160f), flavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These include of the aluminum salts of FD&C dyes extended on an insoluble substrate of alumina hydrate. Additionally, in some embodiments, certified colors can be included as calcium salts.

In some embodiments, natural fruits or plant juice or extracts may be used as the coloring agents. Example include without limitation carrot juice, raspberry juice, blackberry juice, blueberry juice, and beet juice.

### Plasticizer

In some embodiments, the composition may further include plasticizer to modify the texture of the formulation. A texture-modifying agent may include a particulate material. Suitable particulate materials can include, but are not limited to, sucrose, polyols such as sorbitol, xylitol, mannitol, galactitol, lactitol, maltitol, erythritol, isomalt, hydrogenated starch hydrolysates and mixtures thereof, starches, proteins, and combinations thereof. In some embodiments, the particulate material serving as a texture modifying component is selected based on its ability or lack of ability to crystallize the saccharides in the saccharide portion. For example, when isomalt is included in the saccharide portion, sorbitol powder can be added to the composition because it will not cause the isomalt to crystallize. Alternatively, when erythritol is included in the saccharide portion, erythritol powder can be added to the composition because it will cause the erythritol to crystallize. Such particulates can be included in amounts from 5% to 35% w/w of the composition.

### Fats and oils

In some embodiments, a texture-modifying component can include fats, oils, or other hydrophobic materials. Suitable fats can include, but are not limited to, partially hydrogenated vegetable or animal fats, such as coconut oil, corn oil, palm kernel oil, peanut oil, soy bean oil, sesame oil, cottonseed oil, cocoa butter, milk fat, beef tallow, and lard, among others. Suitable hydrophobic materials include chocolate, chocolate crumb, carob coatings, and compound coatings. Such fats, oils, and/or hydrophobic materials can be included in amounts of 1% to 10% w/w of the composition.

In some embodiments, the sensory perception of the texture-modifying component is similar to that of fat, oil, or other hydrophobic materials. For example, a composition including 2.5% fats or oil can provide the same mouth feel perception as a composition including 10%-50% fat as measured by sensory evaluation techniques.

Suitable oils and fats usable in compositions include vegetable or animal fats, such as butter, coconut oil, palm kernel oil, beef tallow, and lard, among others. These ingredients when used may be present in amounts up to about 7%, or up to about 3.5%, by weight of the composition.

In some embodiments, the composition may include edible oil component present in an amount of from about 1% to about 30%, alternatively 1% to 5%, alternatively 5% to 10%, alternatively 10% to 15%, alternatively 15% to 20%, alternatively 20% to 25%, alternatively 25% to 30%, by weight of the composition. In some embodiments, the edible oil component may be present in an amount of from about 0% to about 30%, alternatively 0% to 1%, alternatively 1% to 5%, alternatively 5% to 10%, alternatively 10% to 15%, alternatively 15% to 20%, alternatively 20% to 25%, alternatively 25% to 30%, by weight of the composition. This edible oil component makes up part of the group of ingredients that adjust the taste, texture, and improve the melt and mouth feel of the flavored chewy or gummy confection. For example, in some embodiments, the interaction of the group of highly unsaturated oils with the coconut oil component may creats an improved elasticity within the flavored chewy confection that acts similar to hydrogenated or partially hydrogenated fat. The edible oil component also improves the health characteristic of the flavored chewy confection compositions because it adds monounsaturated and polyunsaturated fats. An example of an edible oil component is a blend of canola, soybean oil, and sunflower oil.

Non-limiting examples of edible oil components acceptable for use in the preferred embodiments include those that have low saturated fat content and high unsaturated fat including monounsaturated and especially polyunsaturated oils. The edible oil component should have no specific flavor and preferably is basically bland or somewhat buttery in taste. The edible oils component can be selected from the following; canola oil, soybean oil, safflower oil, sunflower oil, sesame oil, walnut oil, olive oil, flaxseed oil, chia seed oil, almond oil, corn oil, grape seed oil, peanut oil, other nut oils, and synthesized or reorganized oils, and combinations thereof.

In some embodiment, the edible oil component may have a high level of saturated fats present in an amount of from about 0.3% to about 20%, alternatively 0.3% to 3%, alternatively 3% to 5%, alternatively 5% to 10%, alternatively 10% to 15%, alternatively 15% to 20%, by weight of the composition. Suitable oils having a high level of saturated fats include, but are not limited to, one or more selected from the group consisting of coconut oil, palm oil, fractionated coconut or palm oil, partially hydrogenated coconut or palm oil, fully hydrogenated coconut or palm oil, or any other synthesized or altered edible oils including partially hydrogenated oils and fully hydrogenated oils that have either highly saturated or highly unsaturated fatty acids that when hydrogenated become solid similar to coconut oil in consistency including partially hydrogenated soybean oil, cotton seed oil, palm kernel oil or combination of these edible oils. In one embodiment, the edible oil component comprises coconut oil. This oil component forms a part of the flavor profile and provides a texture to the flavored chewy or gummy confection, and it improves the taste, texture, melt, and mouth feel of the compositions. The blend of the flavor components provides for a great taste, texture, melt and mouthfeel, without the necessity of using partially hydrogenated or fully hydrogenated oils. Any medium heat processed coconut oil can be used.

### Humectant

The glycerin is a humectant and freezing point depressant. It also helps decrease the tendency for granulation and aid in maintaining softness. In some embodiment, glycerin or equivalent material may be employed at a level of from about 1 to about 5% by weight of the final product, e.g., 2 to 3%.

Humectants that can provide a perception of mouth hydration may be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof.

### Bulking agents

Suitable sugar bulking agents include monosaccharides, disaccharides and polysaccharides such as xylose, ribulose, glucose (dextrose), lactose, mannose, galactose, fructose (levulose), sucrose (sugar), maltose, invert sugar, partially hydrolyzed starch and corn syrup solids, and mixtures thereof.

Suitable sugar alcohol bulking agents include sorbitol, xylitol, mannitol, galactitol, lactitol, maltitol, erythritol, isomalt and mixtures thereof. Suitable hydrogenated starch hydrolysates include those disclosed in U.S. Pat. No. 4,279,931 and various hydrogenated glucose syrups and/or powders which contain sorbitol, maltitol, hydrogenated disaccharides, hydrogenated higher polysaccharides, or mixtures thereof. Hydrogenated starch hydrolysates are primarily prepared by the controlled catalytic hydrogenation of corn syrups. The resulting hydrogenated starch hydrolysates are mixtures of monomeric, dimeric, and polymeric saccharides. The ratios of these different saccharides give different hydrogenated starch hydrolysates different properties. Mixtures of hydrogenated starch hydrolysates, such as LYCASIN.RTM., a commercially available product manufactured by Roquette Freres of France, and HYSTAR.RTM., a commercially available product manufactured by SPI Polyols, Inc. of New Castle, Del., are also useful.

### Emulsifiers

The composition may include an emulsifier. The emulsifier may present in an amount of from about 0.001% to about 5%, alternatively 0.001% to 1%, alternatively 1% to 3%, alternatively 3% to 5%, by weight of the composition. In some embodiments, the emulsifier present in an amount of from about 0% to about 5%, alternatively 0.001% to 1%, alternatively 1% to 3%, alternatively 3% to 5%, by weight of the composition.

Example emulsifiers include but not limited to modified corn starch, mono-and diglycerides, and lecithin.

The emulsifier may assist in holding together the fats and water and other components together in a homogeneous composition. In one embodiment, the emulsifier may assist in the formation of a "water and oil" emulsion that creates the smooth texture of the finished product.

### Liquids

Liquids may be used to assist in the flavoring and texture profile of the products. In some embodiments, the composition may include from about 0.001% to about 25% by weight of a fruit or vegetable or combination juice or concentrate component, alternatively 0.001% to 5%, alternatively 5% to 10%, alternatively 10% to 15%, alternatively 15% to 20%, alternatively 20% to 25%, by weight of the composition. The fruit or vegetable or combination juice or concentrate component adds a flavor to the flavored chewy or gummy confection. Any suitable source from the following may used in the embodiments; citrus fruit juices, orchard fruit juices, berry fruit juices, vine fruit juices, decolorized juices, and vegetable juices can be used for this component. The forms can come from juices or concentrates of fruits or vegetables.

In some embodiments, the composition may also include a water component present in an amount of about 0% or greater. The water component adds to the overall texture and melt and chewiness of the flavored chewy or gummy composition. For example, water may be used because of the increase in viscosity of some example compositions. In some embodiments, the composition may contain water from about 1% to about 20%, alternatively 1% to 5%, alternatively 5% to 10%, alternatively 10% to 15%, alternatively 15% to 20%, by weight of the composition.

### Preservatives

Preservatives may be natural or synthetic. Non-limiting examples of suitable preservatives include: sodium benzoate, sodium citrate, sodium phosphate, potassium metabisulfite, sodium metabisulfite, sodium lactate, sodium sulfite, EDTA (ethylenediaminetetraacetic acid), methylparaben, TBHQ, tocopherols, and mixtures thereof. Natural preservatives may include phenols (phenolic acid, polyphenols, tannins), isoflavonoids, organic acids (acetic, lactic, citric), and herb extracts such as extracts of citrus fruits, oregano, thyme, sage, rosemary, clove, coriander, garlic, and onion.

In some embodiments, the composition may include at least about 0% to 2%, by weight of the composition of a preservative component from above, or mixtures thereof.

### Thickening agent

The composition may further include a thickening agent to help with the viscosity of the final product. Some thickening agents are gelling agents. Others act as mechanical thixotropic additives with discrete particles adhering or interlocking to resist strain.

In some embodiments, the thickening agent may be polysaccharides or protein. Example polysaccharides thickening agents include starches, vegetable gums and pectin. Example starch based thickening agents include arrowroot, cornstarch, katakuri starch, potato starch, sago, tapioca and their starch derivatives. Example vegetable gums based thickening agents may include alginin, guar gum, locust bean gum, and xanthan gum. Example protein based thickening agents include collagen, egg whites, furcellaran, and gelatin. Sugar based thickening agent may include agar and carrageenan.

### Methods of making

Processing typically starts with the preparation of a boiled mixture of mixed sugars, which is then blended with the gelling component and processed into shapes by depositing into starch molds. It can also be simply cast onto a slab or cast into rubber molds. The pieces are then held to set and dry. For a general description of this type of process, see Lees and Jackson; Sugar Confectionery and Chocolate Manufacture; 1973 (ISBN 0249 44120 9); pages 226-268.

The method may further include a final processing process in which the final product is prepared. This process can include, for example, extruding, thermoforming, molding, shaping, cutting, and the like, to form the final product in the desired shape. Those skilled in the art are capable of designing a suitable final processing procedure to prepare the final gelled or gummy products, depending on the desired texture (e.g., chewy or gummy) and shape (e.g., cube, square, sheet, animal shaped, etc.).

The embodiments now will be described in more detail with reference to the non-limiting examples that follow.

### EXAMPLES

### Example 1. Low Glycemic Index, Sustained Energy Release, Diabetic Safe, Anti-obesity Gummy Composition

Ingredients: water, 40.2 grams pectin, 160.0 grams trehalose, sodium citrate, coconut oil, 160.0 grams isomaltulose, 410 grams sorbitol syrup, raspberry flavor, citric acid, Lakes red food color
Method: Trehalose, pectin, sodium citrate, and isomaltulose were mixed and dissolved into hot water to provide a first mix. Sorbitol was dissolved into water and the sorbitol solution is then added to the first mix. The total mixture is heated to brix 82. Flavor and citric acid were added followed by the addition of red food color.

### Example 2. Low Glycemic Index, Sustained Energy Release, Diabetic Safe, Gummy Composition

Ingredients: Water, pectin, 160.1 grams trehalose, sodium citrate, coconut oil, 160.3 grams isomaltulose, 470 grams psicose syrup, Passion Fruit flavor, citric acid, Lakes Yellow food color
Method: Trehalose, pectin, sodium citrate, and isomaltulose were mixed and dissolved into hot water to provide a first mix. Psicose syrup was concentrated and added to the first mix. The total mixture was heated until brix 82. Flavor and citric acid were added followed by the addition of food color. The gummy batter was then added to molds and allowed to cool to room temperature.

### Example 3. Low Glycemic Index, Sustained Energy Release, Diabetic Safe, Anti-obesity Gummy Composition

Ingredients: water, pectin, 160.0 grams trehalose, sodium citrate, coconut oil, 160.1 grams isomaltulose, 470 grams tagatose syrup, Candy Green Apple flavor, citric acid, Lakes Green food color
Method: Trehalose, pectin, sodium citrate and isomaltulose were dissolved into hot water to provide a first mix. Tagatose syrup was concentrated and added to the first mix. The total mix was heated until brix 82 was reached. Flavor and citric acid were added followed by the addition of food color. The gummy batter is then added to molds and allowed to cool to room temperature.

### Example 4. Low Glycemic Index, Sustained Energy Release, Diabetic Safe, Anti-obesity Gummy Composition

Ingredients: water, pectin, 160.0 grams trehalose, sodium citrate, coconut oil, 160.1 grams isomaltulose, 235 grams tagatose syrup, 235 grams psicose syrup, Candy Green Apple flavor, citric acid, Lakes Green food color
Method: Trehalose, pectin, sodium citrate, and isomaltulose were dissolved into hot water to provide a first mix. Tagatose syrup and psicose syrup were mixed, concentrated and then added to the first mix. The total mix was heated until brix 82 was. Flavor and citric were added followed by the addition of food color. The gummy batter is then added to molds and allowed to cool to room temperature.

**Example 5.** Low Glycemic Index, Sustained Energy Release, Diabetic Safe, Anti-obesity, Amino Acid Fortified Gummy Composition with added herbal anti-inflammatory.
Ingredients: water, pectin, 160.0 grams trehalose, sodium citrate, coconut oil, 160.0 grams isomaltulose, 5.0 grams L-leucine, 5.0 grams L-isoleucine, 5.0 grams L-valine, 2.2 grams rhizome ginger, 2.2 grams bozwellia, 470 grams psicose syrup, raspberry flavor, citric acid, Lakes red food color
Method: Trehalose, pectin, sodium citrate, isomaltulose, trehalose, leucine, isoleucine, valine, ginger and boswellia were dissolved and mixed into hot water to provide a first mix. Psicose syrup was concentrated and added to the first mix. The total mixture was heated to brix 82. Natural raspberry flavor and citric acid were added followed by the addition of red food color. The gummy batter was then injected to molds and allowed to cool to room temperature.

**Example 6.** Low Glycemic Index, Sustained Energy Release, Diabetic Safe, Anti-obesity, Amino Acid Fortified Gummy Composition with added herbal anti-inflammatory.

Ingredients: water, pectin, 160.0 grams trehalose, sodium citrate, coconut oil, 160.0 grams isomaltulose, 16.5 grams BCAA mix (2:1:1; L-leucine, L-isoleucine, L-valine), 2.2 grams rhizome ginger, 2.2 grams bozwellia, 470 grams psicose syrup, Pear flavor, citric acid, Lakes green food color.

Method: Trehalose, pectin, sodium citrate, isomaltulose, trehalose, BCAA mix, ginger and boswellia were mixed and dissolved into hot water to provide a first mix. Psicose syrup was concentrated and added to the first mix solution. The total mixture was heated to brix 82. Natural raspberry flavor and citric acid were added followed by the addition of red food color. The gummy batter was then injected to molds and allowed to cool to room temperature.

**Example 7.** Low Glycemic Index, Sustained Energy Release, Diabetic Safe, Anti-obesity, Amino Acid Fortified Gummy Composition with added herbal anti-inflammatory.
Ingredients: water, pectin, 100.0 grams trehalose, sodium citrate, coconut oil, 160.0 grams isomaltulose, 60.0 grams trehalose, 16.5 grams BCAA mix (2:1:1; L-leucine, L-isoleucine, L-valine), 2.2 grams rhizome ginger, 2.2 grams bozwellia, 470 grams psicose syrup, Pear flavor, citric acid, Lakes green food color
Method: Trehalose, pectin, sodium citrate, isomaltulose, BCAA mix, ginger and boswellia were mixed and dissolved into hot water to provide a first mix. Psicose syrup was concentrated and added to the first mix. The total mixture was heated until brix 82. Natural raspberry flavor and citric acid were added followed by the addition of red food color. The gummy batter was then injected into molds and allowed to cool to room temperature.

**Example 8.** Low Glycemic Index, Sustained Energy Release, Diabetic Safe, Anti-obesity, Amino Acid Fortified Gummy Composition with added herbal anti-inflammatory.
Ingredients: water, glycerol, pectin, 160.0 grams trehalose, sodium citrate, coconut oil, 160.0 grams isomaltulose, 60.0 grams trehalose, 16.5 grams BCAA mix (2:1:1; L-leucine, L-isoleucine, L-valine), 2.2 grams rhizome ginger, 2.2 grams bozwellia, 470 grams Tagatose syrup, Apple flavor, citric acid, Lakes green food color
Method: Trehalose, pectin, sodium citrate, isomaltulose, BCAA mix, ginger and boswellia were mixed and dissolved into hot water to provide a first mix. Tagatose syrup was concentrated and added to the first mix. The total mixture is heated until brix 82 is reached. Natural raspberry flavor and citric acid were added followed by the addition of red food color. The gummy batter was then added to molds and allowed to cool to room temperature.

**Example 9.** Low Glycemic Index, Sustained Energy Release, Diabetic Safe, Anti-obesity, Amino Acid Fortified Gummy Composition with added herbal anti-inflammatory.
Ingredients: water, glycerol, pectin, 160.0 grams trehalose, sodium citrate, coconut oil, 160.0 grams isomaltulose, 20.5 grams BCAA mix (2:1:1; L-leucine, L-isoleucine, L-valine), 4.2 grams rhizome ginger, 235 grams tagatose syrup, 235 grams psicose syrup, Apple flavor, citric acid, Lakes green food color
Method: Trehalose, pectin, sodium citrate, isomaltulose, trehalose, BCAA mix, and ginger were mixed and dissolved into hot water to provide a first mix. Tagatose syrup and psicose syrup were mixed together, concentrated, and then added to the first mix. The total mixture was heated until brix 82. Natural raspberry flavor and citric acid were added followed by the addition of red food color. The gummy batter was then added to molds and allowed to cool to room temperature.

**Example 10.** Low Glycemic Index, Sustained Energy Release, Diabetic Safe, Gummy Composition for Joint Health.
Ingredients: water, pectin, 40.1 grams N-acetyl glucoseamine, 120.0 grams trehalose, sodium citrate, coconut oil, 160.0 grams isomaltulose, 470 grams psicose syrup, Passion Fruit flavor, citric acid, Lakes Yellow food color
Method: N-acetyl glucoseamine, trehalose, pectin, sodium citrate, and isomaltulose were mixed together to provide a first mix. Psicose syrup was concentrated and then added. The total mixed was heated until brix 82 was reached. Flavor and citric acid were added followed by the addition of food color. The gummy batter was then added to molds and allowed to cool to room temperature.

**Example 11.** Same as 10 with tagatose used in place of psicose.

**Example 12.** Joint and Muscle Health with added B-vitamins. Low Glycemic Index, Sustained Energy Release, Diabetic Safe, Anti-obesity, Amino Acid Fortified Gummy Composition with added herbal anti-inflammatory.
Ingredients: water, glycerol, pectin, N-acetyl glucosamine, 160.0 grams trehalose, sodium citrate, coconut oil, 160.0 grams isomaltulose, 20.5 grams BCAA mix (2:1:1; L-leucine, L-isoleucine, L-valine), 4.2 grams rhizome ginger, 470 grams psicose syrup, 2.2 grams B-vitamin mix, Apple flavor, citric acid, Lakes green food color
Method: N-acetyl glucosamine, trehalose, pectin, sodium citrate, isomaltulose, trehalose, BCAA mix, and ginger were mixed and dissolved into hot water to provide a first mix. Psicose syrup was concentrated and added to the first mix. The total mixture was heated to brix 82. Flavor, vitamin premix, and citric acid were added followed by the addition of food color. The gummy batter was then added to molds and allowed to cool to room temperature.

**Example 13.** Same as 12 but with tagatose syrup replacing psicose syrup.

**Example 14.** Same as 12 but with 235 grams of tagatose and 235 grams psicose syrups mixed together.

### Example 15. Inulin, Calcium Citrate GummyIngredients: Water, pectin, 400 grams tagatose, 202.9 grams calcium citrate, coconut oil, 1430 grams psicose, 95 grams Inulin, citric acid, Passion Fruit flavor, Lakes Yellow food color

Method: Tagatose, pectin, citric acid, and calcium citrate were mixed and dissolved into hot water with pre-dissolved inulin to provide a first mix. Water was used to dissolve psicose which was concentrated to a syrup and added to the first mix solution. The total mixture was heated until brix 83. Flavor and color were added followed by the addition of food color. The gummy batter was then added to molds and allowed to cool to room temperature.

**Example 16.** Same as Example 15 with 0.55/0.45 ratio of maltitol/isomalt replacing the tagatose and psicose.

**Example 17.** Low Glycemic Index, Diabetic Safe, Anti-obesity, Amino Acid Fortified Gummy Composition with added herbal anti-inflammatory. Sweetened with Monk Fruit.

Ingredients: water, pectin, 400 grams tagatose, sodium citrate, coconut oil, 16.5 grams BCAA mix (2:1:1; L-leucine, L-isoleucine, L-valine), 2.2 grams rhizome ginger, 2.2 grams ginger extract, 1600 grams psicose, 5 grams Monk Fruit Extract, Pear flavor, citric acid, Lakes green food color.

Method: Tagatose, pectin, sodium citrate, BCAA mix, ginger and boswellia were mixed and dissolved into hot water to provide a first mix. Water was used to dissolve psicose which was concentrated to a syrup and added to the first mix solution. The total mixture was heated to brix 83. Natural pear flavor and citric acid were added followed by the addition of green food color. The gummy batter was then injected to molds and allowed to cool to room temperature.

**Example 18.** Same as example 17 with 0.55/0.45 ratio of maltitol/isomalt replacing the tagatose and psicose.

**Example 19.** Joint and Muscle Health with added B-vitamins. Low Glycemic Index, Diabetic Safe, Anti-obesity, Amino Acid Fortified Gummy Composition with added herbal anti-inflammatory.
Ingredients: water, glycerol, pectin, N-acetyl glucosamine, xylose, 400 g Tagatose, sodium citrate, coconut oil, 20.5 grams BCAA mix (2:1:1; L-leucine, L-isoleucine, L-valine), 4.2 grams rhizome ginger, 1600 grams psicose, Monk Fruit Extract, 2.2 grams B-vitamin mix, Apple flavor, citric acid, Lakes green food color
Method: N-acetyl glucosamine, xylose, tagatose, pectin, sodium citrate, BCAA mix, monk fruit extract, and ginger were mixed and dissolved into hot water to provide a first mix. Water was used to dissolve psicose which was concentrated to a syrup and added to the first mix solution. The total mixture was heated to brix 83. Flavor, vitamin premix, and citric acid were added followed by the addition of food color. The gummy batter was then added to molds and allowed to cool to room temperature.

**Example 20.** Same as in Example 19 but with 0.55/0.45 ratio of maltitol/isomalt replacing the tagatose and psicose.

**Example 21.** Hair Skins Nails, Diabetic Safe, Anti-obesity, Collagen Fortified Gummy Composition with added herbal and monk fruit extracts.
Ingredients: water, glycerol, pectin, 400 g tagatose, sodium citrate, cacao butter, 60 grams collagen, 10 grams Angelica Sinensis, 1590 grams Psicose, Hair Skin Nails vitamin and mineral mix, Melon Flavor, 5 grams Monk Fruit Extract, citric acid, Paprika orange food color
Method: Tagatose, monk fruit extract, pectin, sodium citrate, collagen, and angelica are mixed and dissolved into hot water to provide a first mix. Water was used to dissolve psicose which was concentrated to a syrup and added to the first mix solution followed by the addition of the hair, skin, and nails vitamin and mineral mix. The total mixture was heated to brix 83. Natural pear flavor and citric acid were added followed by the addition of green food color. The gummy batter was then injected to molds and allowed to cool to room temperature.

**Example 22.** Same as in Example 21 but with 0.55/0.45 ratio of maltitol/isomalt replacing the tagatose and psicose.

### Example 23. Evaluation of the sensory property of the low GI gummy composition

Round table evaluations were performed with 20 test panelists. Equal amount of sugar component was made into gummy compositions. The low GI sugar component (test 1, test 2, and test 3) were made into the test gummy composition 1, 2, and 3. Equal amount of regular sugar component was made into the reference gummy composition. Test 1 included allulose, trehalose, and isomaltulose; test 2 included allulose and tagatose; test 3 included allulose and isomaltulose. The regular sugar component included glucose, sucrose and fructose. The test and reference gummy compositions were the same with the only difference as the sugar component.

The test gummies were tested against the reference samples in a double-blind test for the panelists to determine similarity between the test and references samples. Each panelist was served with gummy samples coded with two-letter codes at room temperature. After each testing, the panelist was asked to rinse with room temperature water and wait for 15 minutes to clear their palates before testing the next sample.

The panelist rated the four basic taste profile (sweet, bitter, sour, metallic), intensity of each taste, the intensities of off-flavors (such as bitter, metallic and acidic) and the temporal profile of the sample (such as lingering after taste and the length of the linger). The length of the linger was rated over a time period of 0 to 120 seconds. The results showed that there is statistically no different between the test low GI gummy composition and reference regular gummy composition in both the taste profile and the temporal profile of the sweetness.

**Example 24.** Evaluation of the sensory property of the low GI sugar component and each individual ingredient.

A low GI sugar component (allulose, trehalose and isomaltulose) was tested against each sugar ingredient with a similar panel and methodology as in Example 23. All four samples were presented as white powdery substance in the same quantity to the test panelists. Each panelist was asked to pour entire sample into the mouth, not swallow down, and let the powder to dissolve for at least 2 minutes inside the mouth. The panelist was then asked the rate the taste profile of each sample. The result showed that the low GI sugar component exhibited significantly high sweetness intensity than each individual sugar samples. The result proved that the ingredients in the low sugar component exhibit significant sweetness synergy.

## Claims

1. A gummy composition, comprising,
a gelling component in a sufficient amount to provide a cohesive gelled product, and
a low glycemic index sugar component having a glycemic index of not more than 35, wherein the gummy composition comprises not less than 50% weight of the low glycemic index sugar component, and wherein the gummy composition is substantially free of D-glucose, D-fructose, D-sucrose, and sugar alcohols,
wherein the low glycemic index sugar component comprises trehalose, isomaltulose and psicose.

2. The gummy composition of Claim 1, further comprising an active component, wherein the active component comprises a vitamin composition, a mineral composition, an amino acid composition, an antioxidant composition, an herbal composition, or an active pharmaceutical ingredient (API) composition.

3. The gummy composition of Claim 1, wherein the low glycemic index sugar component:
(a) is not less than 70% weight of the gummy composition; or
(b) further comprises L-fructose, L-glucose, L-galactose, sorbose, tagatose, D-maltose (1,4- diglucose) or an isomer of D-sucrose (1,2- fructose glucose), raffinose or a combination thereof; or
(c) comprises trehalose, isomaltulose, and psicose, wherein the ratio of trehalose and isomaltulose is from 2:1 to 1:1 by weight; or
(d) comprises D-psicose from about 50% to about 60% by weight.

4. The gummy composition of Claim 1, wherein the low GI sugar component consists essentially of trehalose, isomaltulose and psicose.

5. The gummy composition of Claim 1, wherein the low GI sugar component further comprises N-acetyl glucosamine.

6. The gummy composition of Claim 1, wherein the gummy composition:
(a) comprises less than 0.001%, 0.1% or 1% of non-sugar sweeteners, sugar substitutes, or sugar alcohols; or
(b) is substantially free of D-sucrose, D-fructose, D-maltose, or D-glucose, non-sugar sweeteners, sugar substitutes, or sugar alcohols.

7. The gummy composition of Claim 1, wherein the gelling component comprises gelatin, collagen, starch, pectin, gellan gum, gum Arabic, carrageenans, guar, agar, alginate, locust bean gum, xanthan, or derivatives thereof.

8. The gummy composition of Claim 1, comprising about 0.5% by weight pectin to about 6% pectin and about 2% to about 10% by weight gelatin or collagen based on the total weight of the composition, or comprising about 0.5% by weight pectin to about 6% pectin and about 1% to about 12% by weight starch based on the total weight of the composition.

9. The gummy composition of Claim 1, wherein the gelling component consists essentially of pectin and gelatin, or the gelling component consists essentially of pectin and collagen.

10. The gummy composition of Claim 2, wherein the herbal composition comprises Angelica sinesis, Astragalus, red dates, goji berry, longan berry, Condonoposis Pilosula (Dangshen), ginseng, gandoerma (Linzhi), coix seed, ginger, Bletilla Tuber (Baiji), white Atractylodes rhizome (Baishu), Radix Angelicae Dahuricae (Baizhi), Typhonium Tuber (baifuzi), Radix ampelopsis (bailian), Rhizoma Bletillae (baiji), Rhizoma Polygonati Odorati (yuzhu), Adenophora root (shashen), lily bulb (baihe), mune bean, licorice, ampelopsis root (bailian), white peony root (baishao), Chinese Yam (Rhizoma Dioscoreae, shanyao), poria (fulin), schisandra, mint, aloe, lemon peel, lemon, orange peel, Polygonum Multiflorum (he-shou-wu), ginko, turmeric, gotu kola (*centella asiatica*), ashwagandha, *tribulus terrestris, mucunna pruriens,* guarana, mate (*Ilex paraguariensis*)*,* ephedra (ephedra sinica or ma-huang), ciwujia, rose, lotus, honeysuckle, chrysanthemum, osmanthus fragran, jasmine, dandelion, peach blossom, blueberry, acai berry, raspberry, blackberry, huckleberry, cranberry, strawberry, lingonberry, gooseberry, black or red currants, cloudberry, hawthorn berry, coffee, moca, or extracts or isolates thereof.

11. The gummy composition of Claim 2, wherein the API composition comprises an antibacterial agent, an antifungal agent, a pain or fever reliever, an analgesics, an anti-inflammatory agent, a steroid, a diabetic medication, an antidiarrheal agent, an antiulcer agent, a proton pump inhibitor, a laxative or cathartic agent, a diuretic agent, an antacid, an antihistamine, a decongestant, an antitussive or expectorant agent, a cough suppressant, a cold medicine, a motion sickness medication, a medication for treating alcoholism or opioid dependence, a smoke cessation agent, an anti-anxiety agent, an antidepressant, a mood stabilizer, an antipsychotic agent, a stimulant, a benzodiazepine, an anxiolytic agent, a Z-drug, a melatonergic agent, a barbiturate, a antidepressant, an antipsychotic agent, a cardiovascular agent, an anti-cancer agent, an immune suppressant, a blood thinner, or a combination thereof.

12. The gummy composition of Claim 2, wherein the API composition comprises acetaminophen, aspirin, salicylic acid, ibuprofen, naproxen, cetirizine, diphenhydramine, loratadine, chlorpheniramine, brompheniramine, alimemazine, cyprohetadine, doxylamine, hydroxyzine, promethazine, caffeine, or nicotine.

13. The gummy composition of Claim 1, further comprising sweeteners, food acids, flavoring agents, coloring agents, humectants, bulking agents, fatty acids, triglycerides, emulsifiers, thickeners, cyclodextrin, or and a mixture thereof.

14. The gummy composition of claim 1, wherein the pH of the composition is about 3.7 to about 4.

15. A process for preparing a gummy composition according to claim 1, comprising,
mixing a gelling agent with the active component in hot water to provide a first solution,
mixing low GI sugars in hot water to provide a second solution,
combining the first solution with the second solution to provide a total mixture, wherein the total mixed is heated to brix 82 to provide a gummy base, and
adding food acid into the gummy base to provide a gummy composition.

## Patentansprüche

1. Gummibonbonzusammensetzung, umfassend:
eine Gelierkomponente in einer ausreichenden Menge, um ein kohäsives geliertes Produkt bereitzustellen, und
eine Zuckerkomponente mit einem niedrigen glykämischen Index, die einen glykämischen Index von nicht mehr als 35 aufweist, wobei die Gummibonbonzusammensetzung nicht weniger als 50 Gew.-% der Zuckerkomponente mit einem niedrigen glykämischen Index umfasst, und wobei die Gummibonbonzusammensetzung im Wesentlichen frei von D-Glucose, D-Fructose, D-Saccharose und Zuckeralkoholen ist,
wobei die Zuckerkomponente mit einem niedrigen glykämischen Index Trehalose, Isomaltulose und Psicose umfasst.

2. Gummibonbonzusammensetzung nach Anspruch 1, weiterhin umfassend eine Wirkkomponente, wobei die Wirkkomponente eine Vitaminzusammensetzung, eine Mineralienzusammensetzung, eine Aminosäurezusammensetzung, eine Antioxidanszusammensetzung, eine Kräuterzusammensetzung oder eine pharmazeutische Wirkstoffzusammensetzung (API-Zusammensetzung) umfasst.

3. Gummibonbonzusammensetzung nach Anspruch 1, wobei die Zuckerkomponente mit einem niedrigen glykämischen Index:
(a) nicht weniger als 70 Gew.-% der Gummibonbonzusammensetzung ausmacht; oder
(b) weiterhin L-Fructose, L-Glucose, L-Galactose, Sorbose, Tagatose, D-Maltose (1,4-Diglucose) oder ein Isomer von D-Saccharose (1,2-Fructose-Glucose), Raffinose oder eine Kombination davon umfasst; oder
(c) Trehalose, Isomaltulose und Psicose umfasst, wobei das Verhältnis von Trehalose und Isomaltulose von 2:1 bis 1:1, bezogen auf das Gewicht, beträgt; oder
(d) D-Psicose von etwa 50 Gew.-% bis etwa 60 Gew.-% umfasst.

4. Gummibonbonzusammensetzung nach Anspruch 1, wobei die Zuckerkomponente mit einem niedrigen GI im Wesentlichen aus Trehalose, Isomaltulose und Psicose besteht.

5. Gummibonbonzusammensetzung nach Anspruch 1, wobei die Zuckerkomponente mit einem niedrigen GI weiterhin N-Acetylglucosamin umfasst.

6. Gummibonbonzusammensetzung nach Anspruch 1, wobei die Gummibonbonzusammensetzung:
(a) weniger als 0,001 %, 0,1 % oder 1 % Nicht-Zucker-Süßstoffe, Zuckeraustauschstoffe oder Zuckeralkohole umfasst; oder
(b) im Wesentlichen frei von D-Saccharose, D-Fructose, D-Maltose oder D-Glucose, Nicht-Zucker-Süßstoffen, Zuckeraustauschstoffen oder Zuckeralkoholen ist.

7. Gummibonbonzusammensetzung nach Anspruch 1, wobei die Gelierkomponente Gelatine, Kollagen, Stärke, Pektin, Gellan, Gummiarabikum, Carrageene, Guargummi, Agar, Alginat, Johannisbrotkernmehl, Xanthan oder Derivate davon umfasst.

8. Gummibonbonzusammensetzung nach Anspruch 1, umfassend etwa 0,5 Gew.-% Pektin bis etwa 6 % Pektin und etwa 2 Gew.-% bis etwa 10 Gew.-% Gelatine oder Kollagen, bezogen auf das Gesamtgewicht der Zusammensetzung, oder umfassend etwa 0,5 Gew.-% Pektin bis etwa 6 % Pektin und etwa 1 Gew.-% bis etwa 12 Gew.-% Stärke, bezogen auf das Gesamtgewicht der Zusammensetzung.

9. Gummibonbonzusammensetzung nach Anspruch 1, wobei die Gelierkomponente im Wesentlichen aus Pektin und Gelatine besteht oder die Gelierkomponente im Wesentlichen aus Pektin und Kollagen besteht.

10. Gummibonbonzusammensetzung nach Anspruch 2, wobei die Kräuterzusammensetzung Angelica sinesis, Astragalus, rote Datteln, Gojibeeren, Longanbeere, Condonopsis pilosula (Dang Shen), Ginseng, Ganoderma (Linzhi), Hiobstränensamen, Ingwer, Bletilla-Knolle (Bai Ji), weiße Atractylodes rhizome (Bai Shu), Radix angelicae dahuricae (Bai Zhi), Typhonium-Knolle (Bai Fu Zi), Radix ampelopsis (Bai Lian), Rhizoma bletillae (Bai Ji), Rhizoma polygonati odorati (Yu Zhu), Adenophora-Wurzel (Sha Shen), Lilienzwiebel (Bai He), Munebohne, Lakritz, Ampelopsis-Wurzel (Bai Lian), weiße Päonienwurzel (Bai Shao), chinesischen Yam (Rhizoma dioscoreae, Shan Yao), japanische Aprikose (Fu Ling), Spaltkörbchen, Minze, Aloe, Zitronenschale, Zitrone, Orangenschale, Polygonum multiflorum (He Shou Wu), Ginko, gelben Ingwer, indischen Wassernabel (Centella asiatica), Schlafbeere, Tribulus terrestris, Mucuna pruriens, Guarana, Mate (Ilex paraguariensis), Meerträubel (Ephedra sinica oder Ma Huang), borstige Taigawurzel, Rose, Lotus, Geißblatt, Chrysanthemum, Osmanthus fragran, Jasmin, Löwenzahn, Pfirsichblüte, Heidelbeere, Acaifrucht, Himbeere, Brombeere, Heidelbeere, Moosbeere, Erdbeere, Preiselbeere, Stachelbeere, schwarze oder rote Johannisbeere, Moltebeere, Hagedorn, Kaffee, Moka oder Extrakte oder Isolate davon umfasst.

11. Gummibonbonzusammensetzung nach Anspruch 2, wobei die API-Zusammensetzung ein Antibakterium, ein Antimykotikum, ein Schmerzmittel oder einen Fiebersenker, ein Analgetikum, ein Antiphlogistikum, ein Steroid, ein Diabetesmedikament, ein Antidiarrhoikum, ein Antiulcusmittel, ein Protonenpumpenhemmer, ein Laxativum oder Abführmittel, ein Diuretikum, ein Antazidum, ein Antihistamin, ein abschwellendes Mittel, ein Antitussivum oder Expektorans, ein Hustenmittel, ein Erkältungsmittel, ein gegen Reisekrankheit wirkendes Mittels, ein Mittel zur Behandlung von Alkoholismus oder Opiatabhängigkeit, ein Mittel zur Raucherentwöhnung, ein Antiangstmittel, ein Antidepressivum, ein Phasenprophylaktikum, ein Antipsychotikum, ein Stimulans, ein Benzodiazepin, ein Anxiolytikum, eine Z-Substanz, ein melatonerges Mittel, ein Barbiturat, ein Antidepressivum, ein Antipsychotikum, ein kardiovaskuläres Mittel, ein Antikrebsmittel, ein Immunsuppressivum, einen Blutverdünner oder eine Kombination davon umfasst.

12. Gummibonbonzusammensetzung nach Anspruch 2, wobei die API-Zusammensetzung Acetaminophen, Aspirin, Salicylsäure, Ibuprofen, Naproxen, Cetirizin, Diphenhydramin, Loratadin, Chlorpheniramin, Brompheniramin, Alimemazin, Cyprohetadin, Doxylamin, Hydroxyzin, Promethazin, Koffein oder Nikotin umfasst.

13. Gummibonbonzusammensetzung nach Anspruch 1, weiterhin umfassend Süßstoffe, Lebensmittelsäuren, Geschmacksstoffe, Farbstoffe, Feuchthaltemittel, Auflockerungsmittel, Fettsäuren, Triglyceride, Emulgatoren, Verdicker, Cyclodextrin und/oder ein Gemisch davon.

14. Gummibonbonzusammensetzung nach Anspruch 1, wobei der pH-Wert der Zusammensetzung etwa 3,7 bis etwa 4 beträgt.

15. Verfahren zur Herstellung einer Gummibonbonzusammensetzung nach Anspruch 1, umfassend:
Mischen eines Geliermittels mit der Wirkkomponente in heißem Wasser, um eine erste Lösung bereitzustellen,
Mischen von Zuckern mit einem niedrigen GI in heißem Wasser, um eine zweite Lösung bereitzustellen,
Kombinieren der ersten Lösung mit der zweiten Lösung, um ein Gesamtgemisch bereitzustellen, wobei das Gesamtgemisch bis zu 82 Grad Brix erhitzt wird, um eine Gummibonbonbasis bereitzustellen, und
Zugeben von Lebensmittelsäure in die Gummibonbonbasis, um eine Gummibonbonzusammensetzung bereitzustellen.

## Revendications

1. Composition gommeuse, comprenant :
un composant gélifiant en quantité suffisante pour produire un produit gélifié cohésif, et
un composant sucre à faible indice glycémique ayant un indice glycémique ne dépassant pas 35, la composition gommeuse comprenant au moins 50 % en poids du composant sucre à faible indice glycémique, et la composition gommeuse étant sensiblement exempte de D-glucose, de D-fructose, de D-saccharose et de sucres alcools,
dans laquelle le composant sucre à faible indice glycémique comprend du tréhalose, de l'isomaltulose et du psicose.

2. Composition gommeuse selon la revendication 1, comprenant en outre un composant actif, le composant actif comprenant une composition vitaminique, une composition minérale, une composition d'acides aminés, une composition antioxydante, une composition à base de plantes, ou une composition qui est un principe actif pharmaceutique (PAP).

3. Composition gommeuse selon la revendication 1, dans laquelle le composant sucre à faible indice glycémique :
(a) constitue au moins 70 % en poids de la composition gommeuse ; ou
(b) comprend en outre du L-fructose, du L-glucose, du L-galactose, du sorbose, du tagatose, du D-maltose (1,4-diglucose) ou un isomère du D-saccharose (1,2-fructose glucose), du raffinose ou une combinaison de ceux-ci ; ou
(c) comprend du tréhalose, de l'isomaltulose et du psicose, le rapport du tréhalose et de l'isomaltulose étant de 2:1 à 1:1 en poids ; ou
(d) comprend du D-psicose dans une proportion d'environ 50 % à environ 60 % en poids.

4. Composition gommeuse selon la revendication 1, dans laquelle le composant sucre à faible IG se compose essentiellement de tréhalose, d'isomaltulose et de psicose.

5. Composition gommeuse selon la revendication 1, dans laquelle le composant sucre à faible IG comprend en outre de la N-acétylglucosamine.

6. Composition gommeuse selon la revendication 1, la composition gommeuse :
(a) comprenant moins de 0,001 %, 0,1 % ou 1 % d'édulcorants non-sucres, de substituts de sucre ou de sucres alcools ; ou
(b) étant sensiblement exempte de D-saccharose, de D-fructose, de D-maltose ou de D-glucose, d'édulcorants non-sucres, de substituts de sucre ou de sucres alcools.

7. Composition gommeuse selon la revendication 1, dans laquelle le composant gélifiant comprend de la gélatine, du collagène, de l'amidon, de la pectine, de la gomme gellane, de la gomme arabique, des carraghénanes, de la gomme de guar, de la gélose, de l'alginate, de la gomme de caroube, de la gomme xanthane, ou des dérivés de ceux-ci .

8. Composition gommeuse selon la revendication 1, comprenant environ 0,5 % de pectine à environ 6 % de pectine en poids et environ 2 % à environ 10 % de gélatine ou de collagène en poids relativement au poids total de la composition, ou comprenant environ 0,5 % de pectine à environ 6 % de pectine en poids et environ 1 % à environ 12 % d'amidon en poids relativement au poids total de la composition.

9. Composition gommeuse selon la revendication 1, dans laquelle le composant gélifiant se compose essentiellement de pectine et de gélatine, ou le composant gélifiant se compose essentiellement de pectine et de collagène.

10. Composition gommeuse selon la revendication 2, dans laquelle la composition à base de plantes comprend Angelica sinesis, l'astragale, la datte rouge, la baie de goji, la longane, Condonoposis pilosula (Dangshen), le ginseng, Ganoderma (Linzhi), l'herbe à chapelets, le gingembre, le tubercule de Bletilla (Baiji), le rhizome blanc d'Atractylodes (Baishu), la racine d'angélique dahurica (Baizhi), le tubercule de Typhonium (baifuzi), la racine d'Ampelopsis (bailian), le rhizome de Bletilla (baiji), le rhizome de Polygonatum odoratum (yuzhu), la racine d'Adenophora (shashen), le bulbe de lys (baihe), le mune bean, la réglisse, la racine d'ampélopsis (bailian), la racine de pivoine blanche (baishao), l'igname chinoise (Rhizoma Dioscoreae, shanyao), le poria (fulin), le schisandra, la menthe, l'aloès, le zeste de citron, le citron, le zeste d'orange, Polygonum multiflorum (he-shou-wu), le ginko, le curcuma, le gotu kola (Centella asiatica), l'ashwagandha, Tribulus terrestris, Mucunna pruriens, le guarana, le maté (Flex paraguariensis), l'éphédra (Ephedra sinica ou Ma-Huang), le ginseng sibérien, la rose, le lotus, le chèvrefeuille, le chrysanthème, Osmanthus fragran, le jasmin, le pissenlit, la fleur de pêcher, la myrtille, la baie d'açaï, la framboise, la mûre, la myrtille, la canneberge, la fraise, l'airelle rouge, la groseille à maquereau, le cassis, la groseille rouge, la mûre arctique, la baie de l'aubépine, le café, le moca, ou des extraits ou isolats de ceux-ci.

11. Composition gommeuse selon la revendication 2, dans laquelle la composition PAP comprend un agent antibactérien, un agent antifongique, un agent anti-douleur ou anti-fièvre, un analgésique, un agent anti-inflammatoire, un stéroïde, un médicament pour diabétique, un agent antidiarrhéique, un agent antiulcéreux, un inhibiteur de la pompe à protons, un agent laxatif ou cathartique, un agent diurétique, un antiacide, un antihistaminique, un décongestionnant, un agent antitussif ou expectorant, un médicament contre la toux, un médicament contre le rhume, un médicament contre le mal des transports, un médicament pour traiter l'alcoolisme ou la dépendance aux opioïdes, un agent de sevrage tabagique, un tranquillisant, un antidépresseur, un stabilisateur de l'humeur, un agent antipsychotique, un stimulant, une benzodiazépine, un agent anxiolytique, un Z-médicament, un agent mélatoninergique, un barbiturique, un antidépresseur, un agent antipsychotique, un agent cardiovasculaire, un agent anticancéreux, un immunosuppresseur, un anticoagulant, ou une combinaison de ceux-ci.

12. Composition gommeuse selon la revendication 2, dans laquelle la composition PAP comprend l'acétaminophène, l'aspirine, l'acide salicylique, l'ibuprofène, le naproxène, la cétirizine, la diphénhydramine, la loratadine, la chlorphéniramine, la bromphéniramine, l'alimémazine, la cyprohétadine, la doxylamine, l'hydroxyzine, la prométhazine, la caféine ou la nicotine.

13. Composition gommeuse selon la revendication 1, comprenant en outre des édulcorants, des acides alimentaires, des aromatisants, des colorants, des humectants, des agents de masse, des acides gras, des triglycérides, des émulsifiants, des épaississants, de la cyclodextrine, ou/et un mélange de ceux-ci.

14. Composition gommeuse selon la revendication 1, le pH de la composition étant d'environ 3,7 à environ 4.

15. Procédé de préparation d'une composition gommeuse selon la revendication 1, comprenant :
le mélange d'un gélifiant avec le composant actif dans de l'eau chaude pour produire une première solution,
le mélange de sucres à faible IG dans de l'eau chaude pour produire une deuxième solution,
une combinaison de la première solution avec la deuxième solution pour produire un mélange total, le mélange total étant chauffé jusqu'à un degré Brix de 82 pour produire une base gommeuse, et
l'addition d'un acide alimentaire à la base gommeuse pour produire une composition gommeuse.
